# EUROPEAN PATENT SPECIFICATION

(11) **EP 1 407 742 B1**
(45) Date of publication and mention of the grant of the patent: **04.03.2009**
(21) Application number: 02771751.1
(22) Date of filing: 21.05.2002
(51) Int. Cl.: A61F 13/15, A61F 13/472, A61F 13/551, A61F 13/534

(54) **INTERLABIAL PAD**
INTERLABIAL-POLSTER
SERVIETTE INTERLABIALE

(30) Priority: 22.05.2001 JP 2001152403; 06.08.2001 JP 2001238427
(43) Date of publication of application: 14.04.2004
(73) Proprietor: UNI-CHARM CORPORATION, Shikokuchuo-shi Ehime 799-0111 (JP)
(72) Inventor: MIZUTANI, Satoshi, Mitoyo-gun, Kagawa 769-1602 (JP); YAMAKI, Koichi, Mitoyo-gun, Kagawa 769-1602 (JP); NODA, Yuki, Mitoyo-gun, Kagawa 769-1602 (JP)
(74) Representative: Main, Malcolm Charles
(86) International application number: PCT/JP2002/004891
(87) International publication number: WO 2002/094155

(56) References cited:
- EP-A- 0 985 397
- EP-A2- 1 097 685
- WO-A-97/07763
- WO-A1-94/17115
- WO-A1-98/08475
- WO-A1-98/57610
- WO-A1-99/01093
- WO-A1-99/01096
- WO-A1-99/26575
- WO-A1-99/55272
- JP-A- 10 033 589
- US-A- 5 454 802

## Description

### Background of the Invention

### Technical Field

The present invention relates to an interlabial pad which can be worn closely fitted into between the labia.

### Background Art

Conventionally, a sanitary napkin and a tampon are used generally as sanitary products for the female. Here, there have been great efforts to prevent the leak of blood from gap caused by poor adhesion near the ostium vaginae as for the sanitary napkin. Moreover, as for the tampon, there have been great efforts for relieving the foreign feeling and the discomfort when wearing it and intervaginal fixing trouble due to the nature of the product.

Under such situation, a sanitary product of an interlabial pad has attracted people as a sanitary product featured between the sanitary napkin and the tampon in recent years.

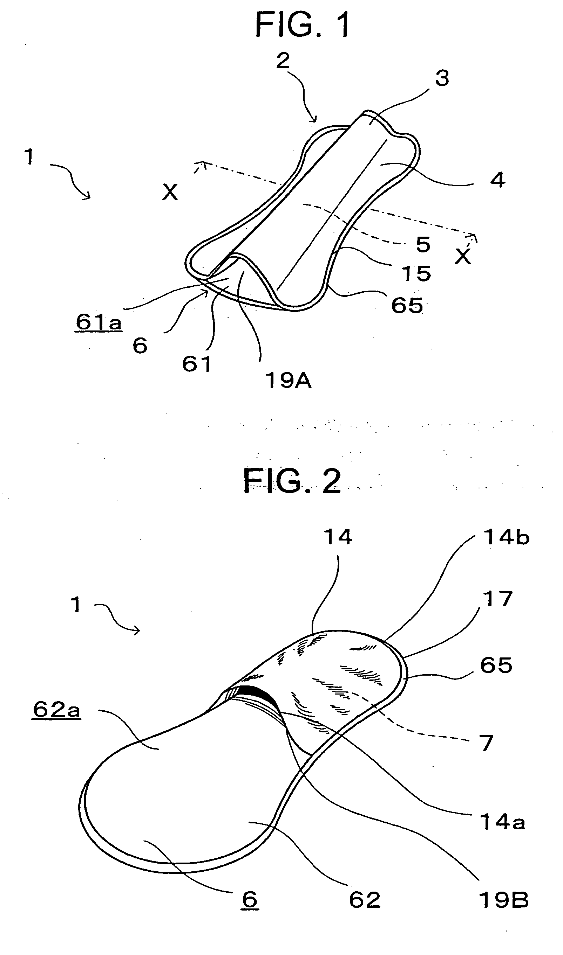

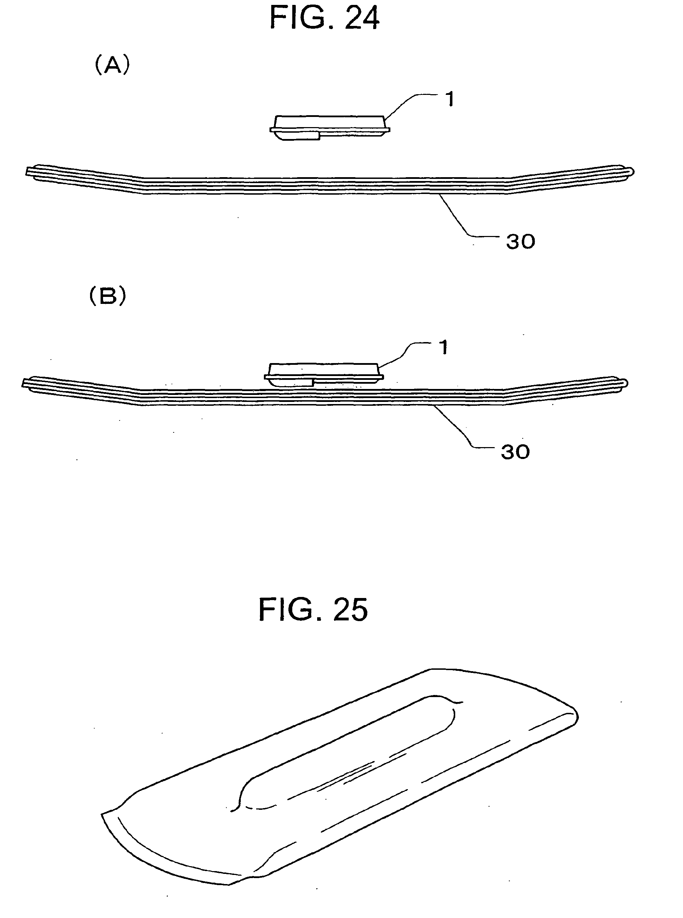

On the other hand, Japan Utility Model Hei 5-18523 (see Fig. 25, which corresponds to Fig. 1 in this Specification) discloses a sanitary napkin, in which an area projected from other area is provided by collectively providing an absorbent body in the central portion of the surface which is brought into contact with the body of a wearer as the product improving the contact state between the sanitary napkin and the body. In the configuration of the sanitary napkin, the projected area is inserted between the labia so that the contact state is improved compared to an ordinal sanitary napkin.

However, in the napkin as described, it is difficult to bring the projected area of the sanitary napkin to a predetermined position. In other words, it is necessary for the wearer to lead skillfully the projected area of the improved sanitary napkin as described above to the predetermined position between the labia by an action of wearing an underwear since the sanitary napkin is fixed to the underwear whereby the napkin is fitted to a body by bringing up the fixed napkin together with the underwear. While wearing the underwear, leading the projected area of the sanitary napkin to the predetermined position is indirect from wearing so that it is more difficult to control it and less precise to put the napkin on the right spot. Also, the projected area is formed by increasing the thickness of the absorbent body, which may cause the wearer to have a strong foreign feeling depending on the thickness.

Although the projected area like that in the example of the related art is not provided, the above-described interlabial pad can obtain the same contact state as the related art may have because of the nature of the product, which is to insert the product between the labia.

However, the interlabial pad is to be put between the labia where is hard to be viewed so that it is not easy to wear it. In addition, if the interlabial pad is not put onto the right position, a great deal of blood leak may cause damages since it is smaller than a sanitary napkin.

Furthermore, the interlabial pad is directly fitted between the labia so that the contact state with the body is easily influenced by the shape of the labia of the wearer. Therefore, it is necessary that the pad be appropriately fitted to any individuals of difference.

European Patent No. EP 0 985 397 discloses a sanitary napkin comprising a liquid-pervious topsheet, a liquid-impervious backsheet and a liquid-absorbent core. The sanitary napkin includes deformable means adapted to deform a transverse middle region of the absorbent core convexly towards a body-facing side of the napkin. The sanitary napkin may be used in combination with an additional napkin wherein the two napkins are bonded together by a layer of adhesive agent.

International Patent Publication No. WO 99/55272 discloses a sanitary napkin comprising a base pad having a liquid-pervious topsheet, a liquid-impervious backsheet and an absorbent core. The sanitary napkin further comprises a tube of absorbent material extending outward from a body-facing side of the base pad. The tube of absorbent material can overlie any suitable portion of the base pad.

European Patent No. 1 097 685 discloses a sanitary napkin including a primary absorbent member and a secondary absorbent member. The primary absorbent member includes a liquid-pervious topsheet, a flexible backsheet and an absorbent core between the topsheet and the backsheet. The secondary absorbent member includes a liquid-pervious topsheet, a liquid-impervious barrier sheet joined to the topsheet and an optional absorbent element positioned between the topsheet and the barrier sheet. The primary absorbent member and the secondary absorbent member are joined together to form a unitary structure.

International Patent Publication No. WO 99/01096 discloses a disposable absorbent article comprising a liquid-pervious topsheet, a backsheet joined to the topsheet and an absorbent core. The disposable absorbent article further comprises means for holding and applying the article to the user's body. The means are located on a garment facing surface and are transversely oriented for insertion of at least one finger for holding and applying the absorbent article.

International Patent Publication No. WO 99/01093 discloses a disposable absorbent article comprising a liquid pervious topsheet, a liquid-impervious backsheet joined to the topsheet and an absorbent core. The disposable absorbent article further comprises means for holding and applying the article to the user's body. The means are located on a garment facing surface and are transversely oriented for insertion of at least one finger for holding and applying the absorbent article.

However, the intertabial pad is positioned between the labia which are difficult to view so that it is not easy to insert it. In addition, if the interlabial pad is not correctly positioned, a great deal of blood leakage may result since the interlabial pad is smaller than a sanitary napkin.

Furthermore, the interlabial pad is directly fitted between the labia so that its contact state with the body is easily influenced by the shape of the labia of the wearer. Therefore, it is necessary that the pad be appropriately fitted to different individuals.

The present invention has been designed to overcome the foregoing problems. An object of the present invention is to provide an interlabial pad, which can be worn in a close contact state by any wearer who has a different shaped labia, in particular, a deep or shallow labia, and which has a configuration so as to achieve sure and sanitary wear.

### Disclosure of the Invention

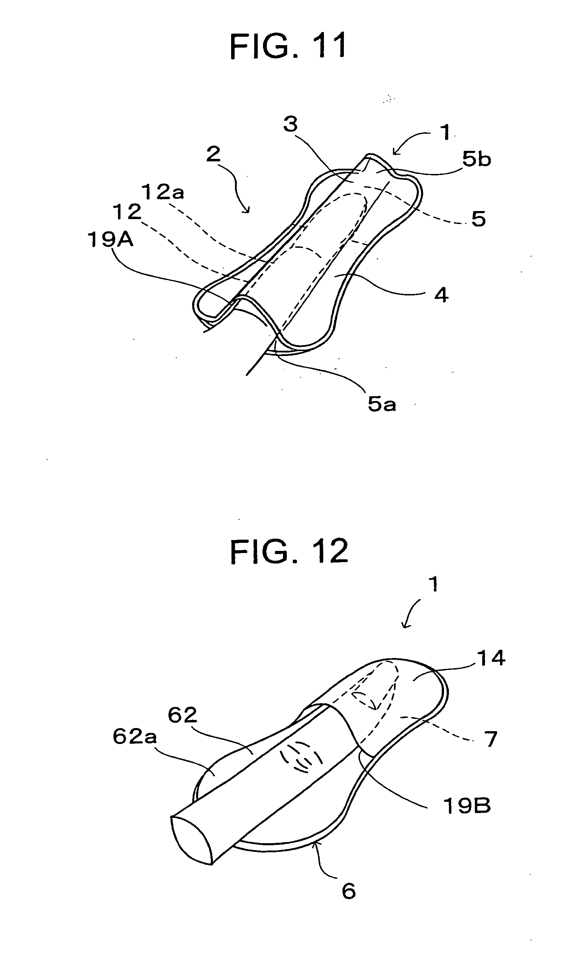

In order to solve the foregoing problems, an interlabial pad according to the present invention is characterized in that a finger insertion opening is provided to the pad so that it can be fitted by skillfully operating a finger on the sensitive finger cushion and that the pad is provided with two choices in the finger insertion opening in accordance with depth of her labia. More specifically, according to the present invention, the interlabial pad is characterized by having a first finger insertion opening formed between a main sheet body and a sub-sheet body and a second finger insertion opening formed between the sub-sheet body and a mini sheet piece. More specifically, the present invention provides the followings.
(1) An interlabial pad for inserting between female labia comprising: a main sheet body composed of a water permeable surface sheet facing a body side and a water permeable back side sheet facing an opposite side of the body side, the main sheet body containing an absorbent body for absorbing body liquid, the absorbent body being enclosed by and between the surface sheet and the back side sheet, the surface sheet and the back side sheet being bonded to each other, and a sub-sheet body composed of a water permeable surface sheet positioned at the body side and a water non-permeable back side sheet facing a clothes side, the sub-sheet body containing an absorbent body for absorbing liquid, the absorbent body being enclosed by and between the surface sheet and the back side sheet, the surface sheet and the back side sheet being bonded to each other; wherein said main sheet body comprises a long convex area formed along a longitudinal direction of the surface side sheet so that a center area of the surface sheet in a lateral direction is formed convex towards the body side ; characterised in that as for characterising clause of claim 1.
   In the interlabial pad according to the present invention, the sheet body including an absorbent body has a dual structure formed with a main sheet body and a sub-sheet body so that blood which has not been absorbed into the main sheet body is absorbed into the sub-sheet body. Therefore, the structure is more effective in preventing blood leakage compared to an interlabial pad with a single structure having the same amount of absorbent body. In addition, since there is a long convex area provided on the surface of the main sheet body, such long convex area is inserted between the labia preventing the gap generated between the labia and the interlabial pad. As a result, blood leakage caused by such gap can be decreased.
   In regards to this, an interlabial pad surface in the related art to be in contact with the body is so flat that the surface to be in contact with the body cannot be closely fitted between the labia because of their concave shape. Therefore, it is possible to have a gap between the labia surface and the interlabial pad such that liquid leakage through the gap may occur. Also, since the sheet body is a single structure, it may not have enough absorbing property.
   However, in the present invention, the main sheet body facing the body side comprises a long convex area, which can be inserted between the labia. Therefore, a gap between the labia surface and the lnterlabial pad can be drastically decreased. Furthermore, the sheet body has a dual structure so that the blood absorbing capacity is improved.
(2) An interlabial pad according to (1); wherein the main sheet body and the sub-sheet body are bonded at each adjacent peripheral edge and are not bonded inside the peripheral edges.
   In the interlabial pad according to the present invention, the main sheet body and the sub-sheet body are bonded only at each peripheral edge and are not bonded (non-stuck) in some area from the inner peripheral edge to the inside. Accordingly, the main sheet body and the sub-sheet body are separated in the middle area so that one of the sheet bodies is not deformed in a similar shape when the other is deformed. In other words, with this structure, deformation of one sheet body is not transmitted to the other sheet body.
   Here, since both sheet bodies are bonded at the peripheral edges, there may be deformation generated in accordance with the bonded state. Specifically, if a wearer having the labia of depth more than the height of the long convex area, the long convex area and surroundings of the main sheet body facing the body side form a long convex area with height sufficient to the depth of the labia of the wearer so as to be inserted between the labia. Only the peripheral edge of the sub-sheet body facing the clothing side which is bonded with the main sheet body is pulled to the body side so that the sub-sheet body as a whole becomes a convex shape facing the opposite side from the body side. Therefore, the sub-sheet body is not to be inserted between the labia.
   As described above, according to the present invention, even if the original height of the long convex area is insufficient for the depth of the labia, the wearer does not feel much foreign feeling since only the main sheet body thickness is inserted between the labia.
   If a wearer having the labia of depth less than the height of the long convex area, a part of the long convex area formed in the main sheet body is inserted between the labia but surrounding area of the long convex area is not inserted between the labia. Hence, the peripheral edge of the sub-sheet body is not pulled towards the body side. Therefore, the sub-sheet body remains almost in the original shape when the interlabial pad is worn.
   Here, "peripheral edge" in the specification is broadly referred to area where the main sheet body and the sub-sheet body are bonded. Each sheet body has more degree of deformation capability since more area of the main sheet body and sub-sheet body may be separable as the "peripheral edge" is positioned closer to the outer periphery of each sheet body.
   In the lateral direction of the interlabial pad according to the present invention, extended areas are provided around the bottom of the long convex area. Hence, the extended areas are bent to cover the pudenda and positioned outside of it when the interlabial pad is worn. With this structure, the above-described extended areas positioned to face the ostium vagina perpendicularly functions as a blocking wall even if a large quantity of menstrual blood leakage in the lateral direction occurs. In addition, boundary areas between the long convex area and the extended areas make bent portions sandwiching the pudenda when the interlabial pad is worn and work as as a moat to receive menstrual blood so as to prevent menstrual blood leakage to the outside even in the case that the long convex area cannot absorb all menstrual blood. It is prefereable that the extended areas are substantially flat so as to perform effectively a function to cover the pudenda.
   As described, in the interlabial pad according to the present invention, an effect of preventing blood leakage is remarkably improved by its distinctive feature, which also improves an effect to preventing the pad from falling off. In other words, since the long convex area provided on the body side surface of the main sheet body of the interlabial pad is fitted closely between the labia, the interlabial pad is tightly fixed to the body of the wearer thereby preventing the whole interlabial pad from falling off the body.
   In respect to the effect to prevent the fall-off, the contact state may be further improved by applying an adhesive agent beforehand to either or both the body side surface of the surface side sheet of the main sheet body and the body side surface of the surface side sheet of the sub-sheet body. As a result, the effect to prevent the fall-off can be further improved.
   When the main sheet body and the sub-sheet body are bonded, it is preferable to provide the bonding area other than a position of bonding area where the mini sheet piece and the sub-sheet body are bonded. Thereby, a portion around the peripheral edge of the interlabial pad where both bonding areas are provided with more stiffness can be prevented so that the interlabial pad keep the flexibility to make a preferable wear feeling.
   In the interlabial pad according to the present invention, at least one of the sleeve portions of the main sheet body or the sub-sheet body is not bonded in the lateral direction so that it makes a sleeve opening to form a finger insertion opening (see Fig. 11) between the main sheet body and the sub-sheet body where a finger can be inserted.
   Since only at left and right side edges of the main sheet body and the sub-sheet body are bonded in the longitudinal direction, a long hollow space is formed between the main sheet body and the sub-sheet body under the long convex area formed on the main sheet body. The long hollow space makes a finger insertion space where a finger can be inserted.
   When both sleeve portions of the main sheet body and the sub-sheet body are non-bonded in the lateral direction, space for finger insertion makes a through hole (like a tunnel) and, when one of the sleeve portions is bonded, space for finger insertion makes a like cave with a closed end.
   In the present invention, "side portion" of the main sheet body or the sub-sheet body in the longitudinal direction includes not only the area corresponding to the peripheral edge of the interlabial pad but also some area surrounding the peripheral edge where the main sheet body and the sub-sheet body can be bonded.
   In the interlabial pad according to the present invention as described, since the main sheet body and the sub-sheet body are bonded to form the finger insertion opening connected to space for finger insertion, the interlabial pad can be temporarily fixed to a fingertip by inserting a finger into the finger insertion opening.
   Then, when a finger is inserted, a fingerprint side of a fingertip from the first joint, where many receptors having a keen sense are scattered can be brought into contact with an opposite face on the back side sheet from the body side face of the main sheet body. The long convex area having an absorbing spot can be precisely led to the ostium vagina of a concave shape by moving the interlabial pad in contact with concave or convex surface structures of the labia. As described, in accordance with the present invention, the interlabial pad can be easily and precisely worn between the labia.
   After wearing, since each surface, between which the top of the long convex area is located, collapses inwards so as to fill the space inside the long convex area, the foreign feeling at the time of wearing the interlabial pad can be drastically diminished.
(3) An interlabial pad according to (2); wherein the main sheet body comprises a plurality of main sheet bodies formed in the longitudinal direction, each of which are bonded together at each longitudinal side edge and at least either sleeve portion in the lateral direction is non-bonded so as to form a finger insertion opening, in which a finger can be inserted.
   In the interlabial pad according to the present invention, there are a plurality of main sheet bodies so that a finger insertion opening is formed between each of the main sheet bodies. Therefore, compared to the above-described (3), a wider selection of finger insertion openings are provided when a wearer inserts a finger such that the wearer can wear the interlabial pad comfortably according to her labia depth. If absorbent bodies are contained respectively in a long convex area and extended areas of a first main sheet body facing the body side, and if absorbent bodies are contained only in long convex areas of a second and third main sheet bodies positioned on the opposite side from the body side, the interlabial pad which does not give a foreign feeling to the wearer can be provided while the menstrual blood absorbance efficiency near the ostium of vagina is improved.
(4) An interlabial pad according to any one of (1) to (3); comprising a mini sheet piece fixed on the back side sheet of the sub-sheet body at the clothes side, the mini sheet piece forming a finger insertion opening between the back side sheet and the mini sheet piece.
   In the interlabial pad according to the present invention as described, the first finger insertion opening and a first finger insertion space connected thereto are formed between the main sheet body and the sub-sheet body, and the second finger insertion opening and a second finger insertion space connected thereto are formed between the sub-sheet body and the mini sheet piece. A wearer having her labia of shallow depth can insert her finger into the first finger insertion opening towards the first finger insertion space and, while she makes her finger cushion touch the opposite side face from the body side face of the back surface side sheet of the main sheet body, lead the long convex area towards the inside between the labia. As a result, only a part of the long convex area, which can be inserted between the labia, can be fitted between the labia.
   On the other hand, a wearer having her labia of deep depth can insert her finger into the second finger insertion opening toward the second finger insertion space and, while she makes her finger cushion touch the opposite side face from the body side face of the back surface side sheet of the sub-sheet body, properly lead the long convex area towards the inside between her labia. Thereby, the whole long convex area can be fitted between the labia.
   As described, in the present invention, the interlabial pad has two kinds of finger insertion openings so that the wearer can provide the interlabial pad suitable for her own labia depth by selecting either finger insertion opening according to the size of her labia when she wears the interlabial pad. Furthermore, a manufacture does not have to make various types of interlabial pads corresponding to individual differences in the labia depth.
(5) An interlabial pad according to any one from (1) to (4); wherein the long convex area is composed of a folded portion formed by folding the main sheet body.
   In the interlabial pad according to the present invention as described, the long convex area may be formed by merely bending the main sheet body itself so that it can be easily deformed by an external pressure.
   On the contrary, if a ready-made projection is simply provided on the body side face of the interlabial pad, the projection may not necessarily fit to all wearers with individual differences in the shape of their labia. For example, if the labia depth of the wearer is more than the height of the projection, the projection can fill only a portion of the inside of the labia and thereby leave space between the top of the projection and the ostium vaginae. On the other hand, if the labia depth of the wearer is less than the height of the projection, the whole projection can be inserted between the labia. However, the portion other than the projection is also inserted between the labia so that the projection with unnecessary thickness is to be inserted between the labia. As a result, wear feeling is notably deteriorated.
   In this respect, in the interlabial pad according to the present invention, the long convex area provided on the main sheet body is formed by merely bending the main sheet body itself so that it can easily deformed according to the labia depth of the wearer. Consequently, the interlabial pad can be used regardless of the differences in the labia depth of the wearers.
   For example, when a wearer having her labia depth less than the height of the long convex area wears the pad, only the long convex area of the main sheet body is fitted between labia. The long convex area is formed by merely bending the main sheet body so that the portion which is not fitted between the labia can be flexibly deformed so as to correspond to the shape near the labia. In this respect, it is remarkably distinctive from the case where the ready-made projection is formed by laminating the absorbent bodies. Also, for a wearer with deep labia depth, the interlabial pad according to the present invention can be flexibly applied. For example, if the neighboring substantially flat area as well as the main sheet body is fitted between the labia, the wearer does not feel foreign feeling since the flat area is deformed so as to be in one body with the long convex area.
   The above-described interlabial pad according to the present invention can be made more applicable to different labia shapes of different individuals If more flexible materials are used to enable the pad to be easily deformed.
(6) An interlabial pad according to any one from (1) to (5); wherein the long convex area is formed with a shorter top length than a bottom length in the longitudinal direction; and wherein the sleeve opening is formed with both side edges sloped from a bottom to a top.
   In the interlabial pad according to the present invention, the longitudinal length at the top of the long convex area is shorter than that at the bottom. In other words, the entrance opening of a long hollow space formed under the long convex area, that is, the finger insertion opening formed between the main sheet body and the sub-sheet body is formed with its both edges sloped from the bottom towards the top that has a shorter length than the bottom. Accordingly, the finger insertion opening is to be provided wider compared to the case where the finger insertion opening is simply formed perpendicular to the main sheet body. Therefore, compared to a finger insertion opening with simply vertical edges to the main sheet body, the finger insertion opening according to the present invention is larger. If both side edges of another finger insertion opening are formed sloping from the top having longer length towards the bottom as opposed to the present invention, finger insertion into the opening may be blocked by the main sheet body. As described, according to the present invention, a smooth and easy finger insertion can be achieved.
   Furthermore, by shortening the top of the long convex area, the contact area of the opposite side face from the body side face of the back side sheet of the main sheet body with the finger cushion of the inserted finger may be decreased when the finger is inserted. Thereby, the friction drag generated between the finger cushion and the inner wall of the long convex area can be decreased such that it can be prevented that the just-set interlabial pad is deviated when the finger is pulled out from the finger insertion opening formed inside the long convex area after the interlabial pad is worn between the labia.
(7) An interlabial pad according to any one from (1) to (6); wherein the long convex area is formed with a shorter top length than a bottom length in the longitudinal direction; and wherein the sleeve opening is formed with both side edges sloped from a bottom to a top.
   In the interlabial pad according to the present invention, the longitudinal cross section of the finger insertion opening and space inside the long convex area is triangular so that it can be easily fitted to the finger inserted to the finger insertion opening and space. As a result, since the interlabial pad is firmly held by the finger inserted inside the long convex area, it is easier to locate the wearing point.
   Also, when wearing an interlabial pad with a long convex area, the long convex area is fitted between the labia so that the labia majora and the labia minora on right and left sides, which usually are closed in contact with each other, are slightly opened. However, since the longitudinal cross sectional area of the long convex area is formed substantially triangular with the top corner fitted to the inmost part of the labia in accordance with the present invention, the surface side sheet is brought into contact with any portions inside the labia minora. Hence, the above-described long convex area, which is capable of absorbing blood, can be closely fitted between the labia of the wearer without a gap so that a function to prevent menstrual blood leakage can be further improved as well as ease in wearing as described above.
   The height of the substantial triangle is preferable to be about 5 to 30 mm, and more preferable to be about 10 to 20 mm. If the height is longer than 30 mm, a gap is easily generated between the substantially flat area of the surface side sheet of the main sheet body and the surface of the pudenda, which may cause leakage of menstrual blood through the gap. Also, if the height is shorter than 5 mm, the long convex area on the main sheet body can not be sufficiently fitted between the labia. Since the contact area with the labia is decreased, it may be more likely for the product to fall off the labia. In addition, the average value of the labia depth of pudenda among Japanese female is about 17 mm so that the more preferable range is from 10 to 20 mm.
   The length of the bottom edge of the substantial triangle of the long convex area in the width direction is preferable to be about 1 to 20 mm and more preferable to be about 2 to 10 mm. If the length is longer than 20 mm, the top corner of the substantial triangle becomes too obtuse so that it may be hard to fit the long convex area between the labia of the female pudenda at the time of wearing the pad. Therefore, it is more likely that wearing becomes troublesome or that a fitted pad may shift from the right position. Also, if the length is shorter than 1 mm, the top corner of the substantial triangle becomes too acute so as to give a foreign feeling to the wearer when she uses the product.
   In this respect, in the present invention, the long convex area is formed within the preferable range so that the interlabial pad is formed to give the wearer an excellent wear feeling with high degree of contact to the labia.
   In this Specification, "longitudinal cross section" means a cross section vertical to the surface forming the interlabial pad and a cross section along the direction substantially vertical to the finger insertion direction.
(8) An interlabial pad according to any one from (1) to (7), wherein a longitudinal cross sectional area of the long convex area in the lateral direction is at least 1 cm².
   In the interlabial pad according to the present invention, the longitudinal cross sectional area of the above-described long convex area is large enough for a female in general to insert her finger so that she can easily insert her finger into and remove it out of the inside of the long convex area. Thereby, wearing time can be shortened and it is less likely that the fitted interlabial pad may be shifted from the right position.
(9) An interlabial pad as claimed in any one from (1) to (8); wherein a longitudinal cross sectional area of the long convex area becomes continuously decreased as the area is taken from one end to the other end along the longitudinal direction.
   In the interlabial pad according to the present invention, since the finger , insertion space becomes smaller towards the other end as the finger has a longitudinal cross section area smaller towards the fingertip, the inserted finger fits well to the inner wall of the long convex area. Thereby, since it can prevent the finger from playing in the long hollow space, the finger may have a contact area with the back side sheet of the main sheet body so that a large amount of contact area can be obtained. Therefore, while the interlabial pad is being worn, the concave shape ostium vagina can be located precisely by sensing the concave and convex of the labia via over the main sheet as the interlabial pad is in contact with the labia.
   In the present invention, "to become smaller substantially-continuously" means to become gradually smaller on the average and, as long as it becomes gradually smaller as a whole, a part of the area may become larger or stay equal.
(10) An interlabial pad according to any one from (1) to (9); wherein the interlabial pad is a sanitary-napkin-coexisting-interlabial pad that is used together with a sanitary napkin.
   In the interlabial pad according to the present invention as described, even if it is used together with a sanitary napkin, a negative effect on the wear feeling may not arise and it may be less likely to suffer from rash or stuffy feeling.
   In other words, some sanitary napkin users use several pieces of napkins layered together when they have a large quantity of menstrual blood. However, they may feel uncomfortable because of stiffness of the napkins, which further affect the external appearance. Also, the layered sanitary napkins are put on one after another even near the ostium vaginae where the layered napkins are not needed, which causes rash and stuffy feeling.
   In this respect, with the present invention, the sanitary product is layered only on the labia and its surroundings so that there is not a negative effect on the wear feeling and the external appearance. Also, rash and stuffy feeling in the buttock and its surrondings can be decreased.
   Furthermore, in the case of replacement, it is possible to change only the interlabial pad without changing the sanitary napkin according to the present invention. Therefore, the wearer does not have to carry around the sanitary napkins which are large enough to be noticed. The sanitary napkins herein may include an absorption sheet for vaginal discharge as well as a napkin sold for absorbing menstrual blood.
(11) An interlabial pad according to any one from (1) to (10); wherein the interlabial pad comprises an incontinent-interiabial pad for incontinence.
   According to the interlabial pad of the present invention, the pad can be used for incontinence absorb pad. Since both ostium vaginae where the menstrual blood is discharged and urethral meatus where urine is discharged are located between labia, the interlabial pad of the present invention may be used between labia to absorb urine.
   As described hereinbefore, the pad of the present invention can absorb urine around labia, especially around the urethral meatus and is useful for the absorbing pad for incontinence, especially for a light incontinence.
(12) An interlabial pad according to any one from (1) to (10); wherein the interlabial pad comprises a vaginal-discharge interlabial pad for absorbing vaginal discharge.

In accordance with the present invention, the interlabial pad can be used for the pad of absorbing the vaginal discharge. The interlabial pad is used by being inserted between labia and can absorb the excretion (vaginal discharge) other than the menstrual blood from ostium vaginae (for absorbing the vaginal discharge).

As described above, since the pad can absorb the vaginal discharge in order to decrease the wearer's discomfort, it is useful for the user who is not menstruating.

Each interlabial pad may be wrapped individually so that an individually-wrapped interlabial can be carried independently for use. Hence, as opposed that a plurality of the interlabial pad are wrapped in the same wrapping container, it is cleaner and more convenient to handle each pad.

The interlabial pad may be wrapped without being aligned to the wrapping container. Therefore, the wrapping body is formed so that the first finger insertion opening formed between the main sheet body and the sub-sheet body and the second finger insertion opening formed by the mini sheet piece are to be opened towards the wearer when the wearer opens the wrapping container. Therefore, the wearer's opening direction and finger insertion direction can be made in the same direction so that the wearer can insert the finger easily.

Furthermore, by changing the folding state of the interlabial pad to be wrapped or by indicating the opening direction through drawing a design or character, the wearer can easily select a finger insertion opening according to her own labia depth even if the interlabial pad is individually wrapped according to the present invention.

The positions of the first finger insertion opening formed between the main sheet body and the sub-sheet body and the second finger insertion opening formed by the mini sheet piece can be recognized at a glance without opening the wrapping container. Therefore, the wearer can perform finger insertion to the proper finger insertion opening more easily according to her own labia depth.

In this case, through providing the above-described design and character with the house mark or the company name, in addition to the above-described effect (indication of the finger insertion opening), an advertisement effect and a quality assurance effect can be expected.

### Brief Description of the Drawings

Fig. 1 is a schematic perspective view showing the top face (body side surface) of an interlabial pad according to the embodiment;
Fig. 2 is a schematic perspective view showing the bottom face (opposite side surface to body) of the interlabial pad according to the embodiment;
Fig. 3 is an illustration showing the interlabial pad according to the embodiment to which a plurality of mini sheet pieces are attached;
Fig. 4 is a cross section of the interlabial pad according to the embodiment taken along the line X - X shown in Fig. 1;
Fig. 5 is an explanatory illustration for describing a long convex area of the interlabial pad according to the embodiment;
Fig. 6 is an explanatory illustration for describing that the longitudinal cross sectional areas of the hollows on both edges of the long convex area are different;
Fig. 7 is an explanatory illustration for describing the whole girth inside a second finger insertion opening of the mini-sheet attached to the interlabial pad according to the embodiment;
Fig. 8 is an illustration showing the state where the mini sheet piece attached on the interlabial pad according to the embodiment has a length of 10 % or more in the longitudinal direction;
Fig. 9 is an illustration showing the unbonded position in the back surface side of the mini sheet piece attached on the interlabial pad according to the embodiment;
Fig. 10 is an explanatory illustration for describing the attachment position of the mini-sheet on the interlabial pad according to the embodiment;
Fig. 11 is an explanatory illustration for describing the state when inserting the forefinger from the first finger insertion opening to the hollow space at the time of using the interlabial pad according to the embodiment;
Fig. 12 is an explanatory illustration for describing the state when inserting the forefinger to the second finger insertion opening at the time of using the interlabial pad according to the embodiment;
Fig. 13 is an explanatory illustration for describing that the first finger insertion opening and the second finger insertion opening face towards the same direction;
Fig. 14 is an illustration showing the state when wearing the interlabial pad between the labia according to the embodiment;
Fig. 15 is a front cross section showing the state of the mini sheet piece after wearing the interlabial pad according to the embodiment;
Fig. 16 is an explanatory illustration showing the state when removing the interlabial pad by pulling the mini sheet piece according to the embodiment;
Fig. 17 is a cross section showing the wearing state of the interlabial pad having a ready-made projection;
Fig. 18 is a cross section showing the state when the interlabial pad according to the embodiment is used by a wearer with a short labia depth;
Fig. 19 is a cross section showing the state when the interlabial pad according to the embodiment is used by a wearer with a long labia depth;
Fig. 20 is an illustration showing the state of experiment on measurement of separation force of an adhesive;
Fig. 21 is an illustration showing the state of experiment on measurement of shearing strength of the adhesive;
Fig. 22 is an illustration showing the state where the interlabial pad according to the embodiment is folded and wrapped individually;
Fig. 23 is an illustration showing the state where the interlabial pad according to the embodiment is individually wrapped in a wrapping container to which a character is applied near its opening section;
Fig. 24 is an illustration showing the state where the interlabial pad according to the embodiment is used together with a sanitary napkin;
Fig. 25 is an illustration showing an example of the state of a sanitary napkin of the related art having a ready-made projection;
Fig. 26 is an illustration showing an example of the state of a incontinence support pad of the related art having a finger insertion opening; and
Fig. 27 is an illustration for describing an example of the state of finger insertion in the incontinence support pad of the related art having the finger insertion opening.
Fig. 28 is an illustration for describing a size of the interlabial pad along the lateral direction.

### Best Mode of Carrying Out the Invention

Now, the interlabial pad according to the present invention will be described by referring to the drawings.

### [Basic Structure]

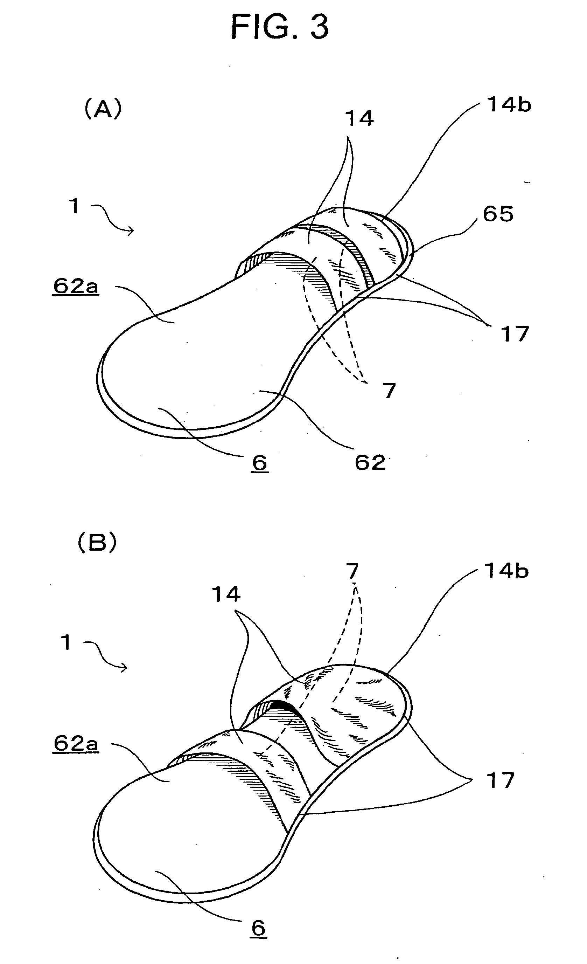

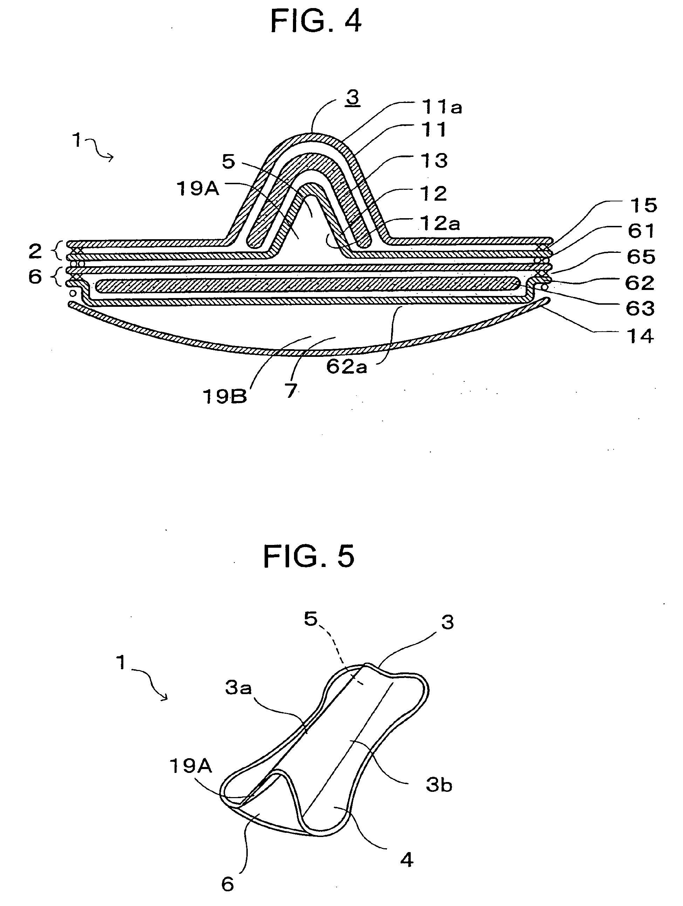

First, the configuration of an interlabial pad 1 according to the embodiment will be described. The interlabial pad 1 according to the embodiment is formed comprising a main sheet body 2, a sub-sheet body 6, a mini sheet piece 14 attached on the sub-sheet body 6 on the opposite side to the body. Fig. 1 is a schematic perspective view showing the body side of the interlabial pad 1 according to the embodiment and Fig. 2 is a schematic perspective view showing the opposite side from the body side of the interlabial pad 1 according to the embodiment. Fig. 3 is an illustration showing other embodiment of the mini sheet piece 14 attached on the opposite side from the body side of the interlabial pad 1 according to the embodiment. Fig. 4 is a cross section of the interlabial pad 1 according to the embodiment taken along the line X-X in Fig. 1.

As shown in Fig. 1, on the body side to the body of the main sheet body 2, a long convex area 3 formed by crumpled portion generated by folding the main sheet body 2 is provided in the longitudinal direction on the main sheet body 2 roughly in the center in the lateral direction. Then, a substantially flat area 4 is continuously provided in the area spread on both sides of the long convex area 3 in the lateral direction. The main sheet body 2, as shown in Fig. 4, is formed as one body by bonding a surface side sheet 11 and a back side sheet 12 in a peripheral edge 15 so that an absorbent body 13 included in the above-described long convex area 3 is sealed inside.

Subsequently, the above-described sub-sheet body 6, as shown in Fig. 4, is formed as one body by bonding a surface side sheet 61 and a back side sheet 62 in a peripheral edge 65 so that an absorbent body 63 is sealed inside. It is preferable for the absorbent body 63 not to be sandwiched in the peripheral edge 65 where the sheets are bonded. For example, by bonding only the surface side sheet 61 and the back side sheet 62 and sealing the absorbent body 63 in the closed area in the peripheral edge 65, hardening of the peripheral edge 65 caused by sandwiching the absorbent body 63 in the peripheral edge 65 can be avoided. Thereby, more preferable wear felling can be achieved. The dimension of the absorbent body 63 may be about that of the interlabial pad 1 or, in order for the absorbent body 63 not to be sandwiched in the above-described peripheral edge 65, it may be provided smaller beforehand so as to be able to provide a gap of 2 to 10 mm from the contour of the interlabial pad 1.

The bonding of the surface side sheet 11 and the back side sheet 12 in the main sheet body 2 and that of the surface side sheet 61 and the back side sheet 62 in the sub-sheet body 6 are multiplied by a heat embossing adhesive and / or hot melting adhesive. Also, the absorbent body 13 is pasted to each of the surface side sheet 11 and the back side sheet 12 to prevent the interlayer separation therefrom, while the absorbent body 63 is pasted to each of the surface side sheet 61 and the back side sheet 62 to prevent the interlayer separation therefrom.

The main sheet body 2 and the sub-sheet body 6, as shown in Fig. 1, are bonded each other in the peripheral edge 15 and the peripheral edge 65 except for both sleeve openings of the long convex area 3, and inside the peripheral edge 15 and the peripheral edge 65 are not bonded. Therefore, a finger insertion opening 19A capable that a finger can be inserted into and a hollow part 5 to be continuing from the finger insertion opening are formed between the inside of the long convex area 3 and a body side surface 61 a in the surface side sheet 61 of the sub-sheet body 6. Also, as shown in Fig. 2, the back side sheet 62 of the sub-sheet body 6 and the mini sheet piece 14 are bonded at a bonding area 17 in the peripheral edge 65 except for one of the sleeve portions 14a of the mini sheet piece 14 and the inside from the peripheral edge 65 is not bonded. Therefore, a finger insertion opening 19B capable that a finger can be inserted into and a hollow part 7 to be a finger insertion space continued therefrom are formed between the opposite side face 62a from the body side face of the back side sheet 62 and the mini sheet piece 14. It is possible to provide a plurality of mini sheet pieces 14. In this case, the number of the bonding areas 17 on left- and right-hand sides increases on the back side sheet 62 in the longitudinal direction according to the number of the pieces. For example, if there are two mini sheet pieces 14, two bonding areas are to be provided on each side.

As described, in the case where a plurality of the mini sheet pieces 14 are provided, it is not necessary for all the mini sheet pieces 14 to be in the same shape as shown in Fig. 3(A). For example, as shown in Fig. 3(B), each of the mini sheet pieces 14 may be in a different shape. Thereby, it can prevent blood from sticking to the finger and the amount of materials used for the mini sheet piece 14 can be decreased.

### [Long Convex Area]

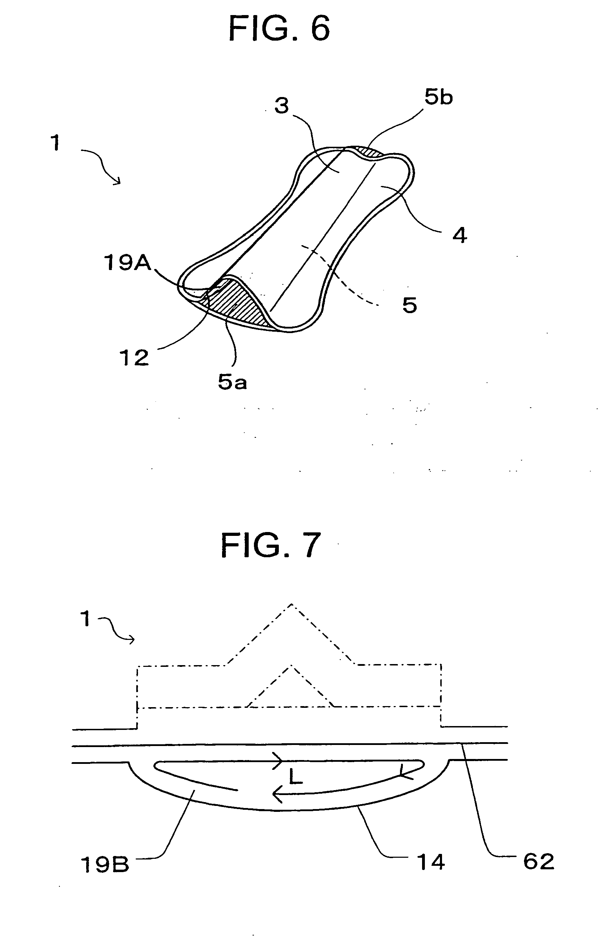

Next, the shape of the long convex area 3 according to the embodiment will be described. Fig. 5 is an explanatory illustration for describing the long convex area 3 of the interlabial pad 1 according to the embodiment. Fig. 6 is an explanatory illustration for describing that the longitudinal cross sectional areas of the hollow part at both ends of the long convex area 3 of the interlabial pad 1 according to the embodiment are different.

As shown in Fig. 5, the long convex area 3 forming a first finger insertion opening 19A is formed in such a manner that the length of the top 3a is shorter than that of the bottom 3b in the longitudinal direction. Hence, in the finger insertion opening 19A of the long convex area 3, the edges on both sides are formed to be sloping from the bottom towards the top. Therefore, a wearer can insert the finger inside the finger insertion opening 19A without an interruption by the edges on both sides and pass through under the top 3a into the hollow part 5. Having the short top 3a means a decrease in the contact area of the finger cushion of inserted finger. In other words, the friction drag generated between the finger and the inner wall of the long convex area 3 is to be decreased when pulling out the finger from the hollow part 5 after wearing the interlabial pad 1 between the labia. As a result, it can drastically decrease the position shift of the interlabial pad 1 after wearing.

Also, the finger insertion opening 19A is substantially triangular and the longitudinal cross sectional area of the hollow part 5 continued therefrom in the lateral direction is 1 cm² or more. With this structure, the finger insertion opening 19A can be maintained to be wide-open to some extent.

The longitudinal cross sectional area of the long convex area 3 in the lateral direction, as shown in Fig. 6, is formed in such a manner that one of the end 5b from the other end 5a becomes sub-continuously smaller in the longitudinal direction. With this structure, the shape of the finger in which the longitudinal cross sectional area becomes smaller towards the fingertip fit to the shape of the hollow part 5. Therefore, the fingertip can be easily kept in contact with the opposite side surface to the body 12a (see Fig. 4) of the back side sheet 12 of the main sheet body 2.

### [Mini Sheet Piece]

Next, the attachment state of the mini sheet piece 14 will be described. In the embodiment, as shown in Fig. 2, the mini sheet piece 14 is bonded with the back side sheet 62 by the bonding area 17 in the outer edge of the back side sheet 62 and the area from the outer edge to the inside is not bonded. Hence, the mini sheet piece 14 is attached over the area from one side of the back side sheet 62 to the other side thereby forming a hollow part 7 to be a second finger insertion space over the area from one side to the other side.

In the embodiment, one of the sleeve portions 14b of the mini sheet piece is bonded with the back side sheet 62 in the lateral direction. However, it can be also prepared unbonded. In this case, the above - described hollow part 7 becomes a elongated space with both open ends (like a tunnel).

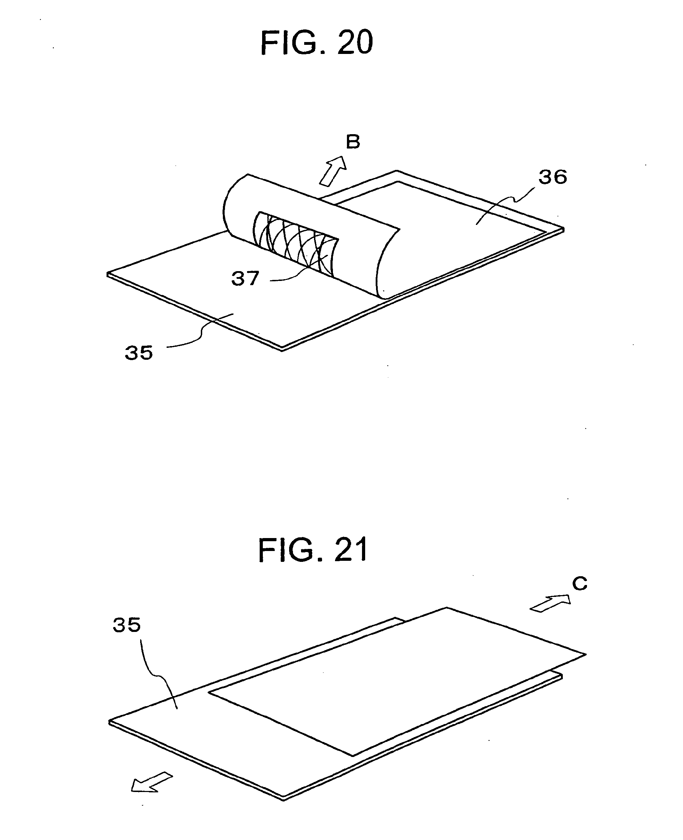

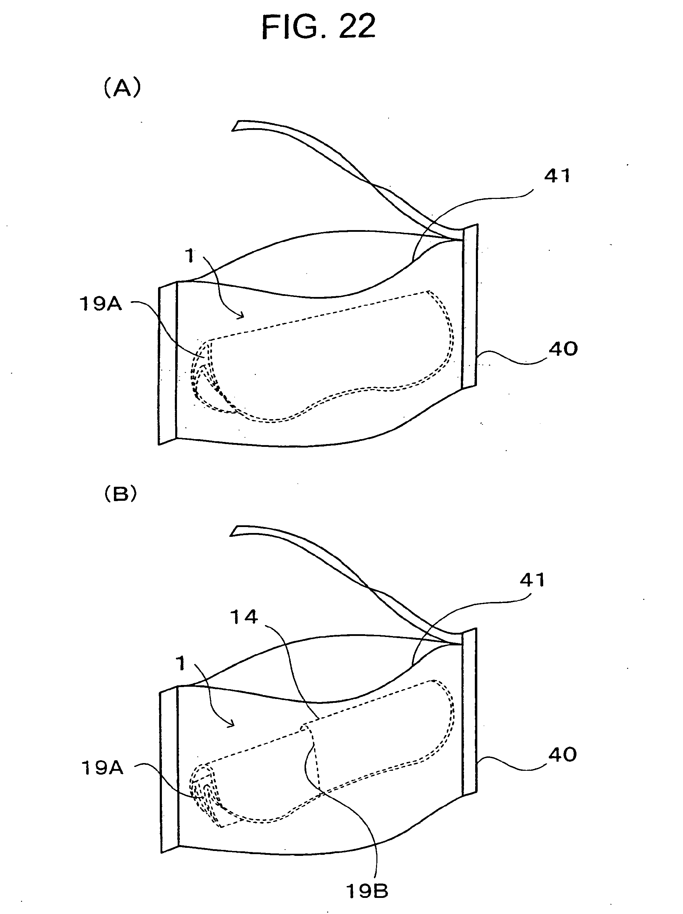

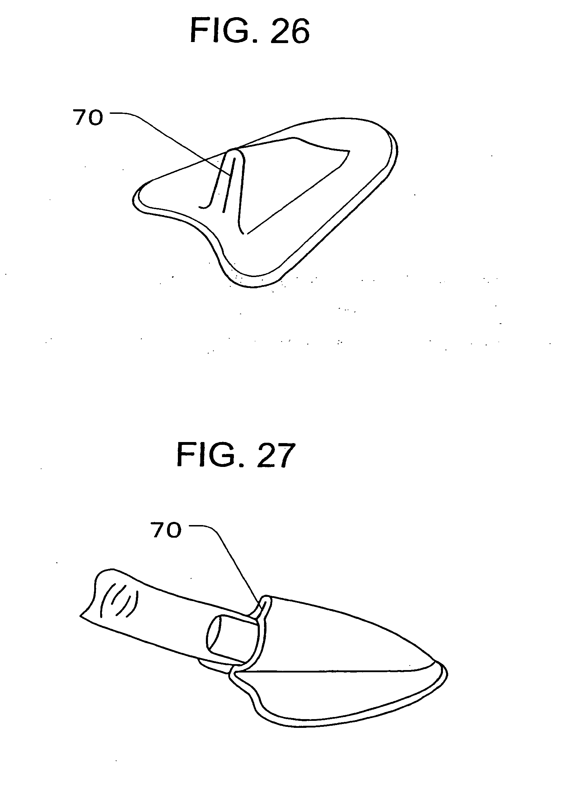

In the interlabial pad 1 according to the embodiment, the mini sheet piece 14 is attached so that the finger insertion opening 19B has an aperture large enough for the fingerbreadth in the direction of the finger nail width. Thereby, the flat-shaped fingertip may be inserted and its flat face may contact the sheet surface without being tilted against the sheet surface. In this respect, compared to an incontinence support pad of the related art (JP Patent Hei 6-506368), the easiness of finger insertion is remarkably improved. In other words, in the incontinence support pad of the related art, a finger opening 70 (code 76 in the above-described Application) is closed (see Fig. 26, which corresponds to Fig. 20 in the above-described Application) in the normal state. Therefore, first, a wearer inserts a finger in the direction at a right angle to the incontinence support pad (see Fig. 27, which corresponds to Fig. 22 in the above-described Application) and then turns the finger so that the finger cushion can be faced to the incontinence support pad side.

On the contrary, in the interlabial pad 1 according to the embodiment, unlike the incontinence support pad of the related art in which the aperture of the fingerbreadth is secondarily formed in the direction of the surface of the back side sheet, the finger insertion opening 19B which is suitable for finger insertion is primarily formed so as to be able to insert the finger naturally. Therefore, the wearer of the interlabial pad 1 can specify the direction of the finger insertion. As a result, the finger cushion naturally detects the wearing point so that precise wearing of the pad between the labia can be more easily achieved.

Fig. 7 is a sectional cross section of a part of the interlabial pad 1 in the lateral direction taken out from the interlabial pad 1 for specifically describing "the whole girth inside the second finger insertion opening 19B". In Fig. 7, the part unnecessary for describing the length of "the whole girth inside the second finger insertion opening 19B" is shown by an alternate long and short dash line. "The whole girth inside the second finger insertion opening 19B" is a distance denoted by "L" in Fig. 7.

The whole girth inside the above-described second finger insertion opening 19B is preferable to be 30 to 120 mm, and more preferable to be 40 to 80 mm. When the whole girth inside the above-described second finger insertion opening 19B is shorter than 30 mm, the second finger insertion opening itself becomes small causing a difficulty in putting the finger in and out. On the other hand, when it is longer than 120 mm, the interlabial pad 1 can not be fixed to the finger. Therefore, it becomes harder for the finger cushion to be surely in contact with the sheet surface, which causes a problem when wearing. Accordingly, the length "L" in the embodiment is about 40 mm.

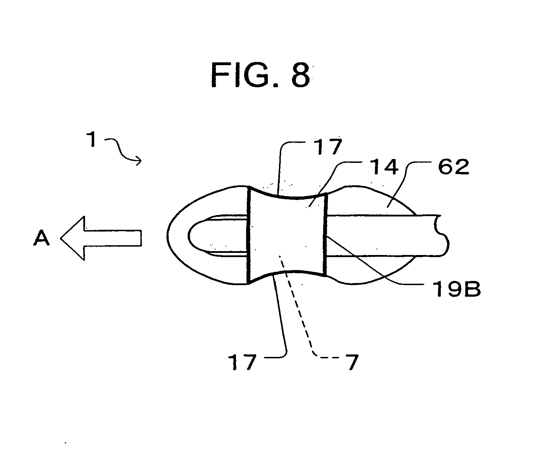

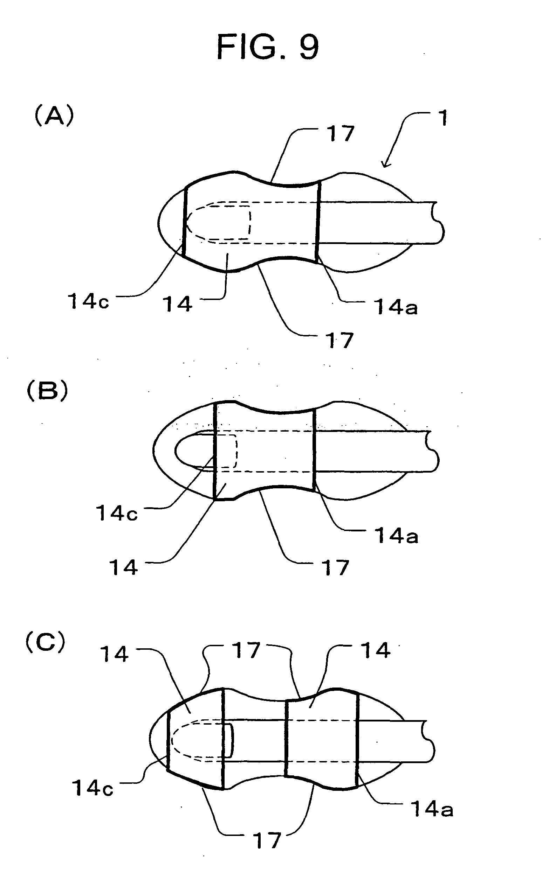

Fig. 8 is an illustration showing the state where the mini sheet piece 14 attached on the interlabial pad 1 according to the embodiment has a length of 10 % or more in the longitudinal direction. Fig. 9 is an illustration showing the position of the nonbonded part in the back side sheet 6 of the mini sheet piece 14 attached to the interlabial pad 1 according to the present invention.

In the embodiment, as shown in Fig. 2, the nonbonded part of the mini sheet piece 14 and the back side sheet 62 is only one of the sleeve portions 14a which forms the second finger insertion opening 19B. However, the other sleeve portion 14b can be also left non-bonded. In this case, the length of the mini sheet piece 14 is preferable to be 10 % or more in the longitudinal direction, more preferable to be 10 to 80 %, and even more preferable to be 30 to 60 %. By having the length as described, there is no chance for the finger once being inserted to the second finger insertion opening 19B to be slipped out therefrom or for the finger to play inside the hollow part 7. Therefore, the finger cushion can be kept facing the sheet surface of the back side sheet 62. Also, as shown in Fig. 8, it is clear that the direction of finger insertion can be provided in "A" direction. In regard to this, "the 10 % or more length of the mini sheet piece 14" serves to indicate the direction of the finger insertion in the interlabial pad 1 according to the present invention.

In the case where a second non-bonded part 14c is provided in addition to the non-bonded part 14a forming the second finger insertion opening 19B, the tip of the finger may be exposed therefrom and may be in contact with blood when wearing the interlabial pad 1. In this respect, as shown in Fig. 9(A), by providing the second nonbonded part 14c in the position where the fingertip of the wearer is entirely covered, the finger can be kept unexposed so as to be in sanitary condition as shown in Fig. 9(B).

Furthermore, as shown in Fig. 9(C), even in the case with a plurality of mini sheet pieces 14 are attached and there are a plurality of the nonbonded parts, exposure of the finger can be prevented in the same manner if the second nonbonded part 14c is provided in the endmost.

### [Bonding Position of the Mini Sheet Piece]

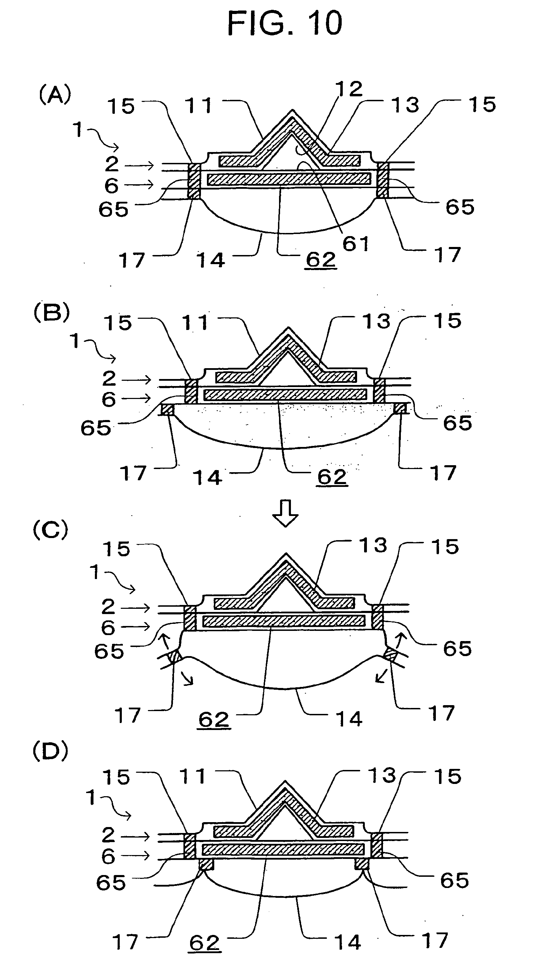

Next, the bonding condition of the main sheet body 2, the sub-sheet body 6, and the mini sheet piece 14 according to the embodiment will be described. As shown in Fig. 10(A), when the bonding area 17 between the mini sheet piece 14 and the back side sheet 62 is fixed together in the same position where the peripheral edge 15 as the bonding area between the surface side sheet 11 and the back side sheet 12 of the main sheet body 2, and the peripheral edge 65 as the bonding area between the surface side sheet 61 and the back side sheet 62 of the sub-sheet body 6 are bonded together, the outer edge side portion of the interlabial pad 1 becomes hard thereby influencing the wear feeling. This can be avoided by positioning and fixing the mini sheet piece 14 with the bonding area 17 in the area other than the peripheral edge 65.

However, as shown in Fig. 10(B), when the bonding area 17 is positioned more outside of the peripheral edge 65 than the peripheral edge 65, as shown in Fig. 10(C), friction is generated by a movement in accordance with the action of the wearer so that it is considered to be possible that the wearer may be irritated.

As described, when positioning, as shown in Fig. 10(D), it is preferable that the peripheral edge 15 and the bonding area 17 are positioned with some distance form each other and the bonding area 17 is positioned inner side than the peripheral edge 65.

When attaching the mini sheet piece 14, pressure sensitive hot melt, thermal sensitive hot melt and the like can be used as an adhesive and can be applied on the whole surface, or in line, spiral, dots and the like.

The mini sheet piece 14 may be cut beforehand so as to fit with the attaching part as described. Also, if the bonding area is positioned in a different position from other sheets, it may be cut together with other sheets.

### [Shape of the Interlabial pad]

The shape of the interlabial pad 1 according to the embodiment may be in any shapes such as elliptic-shape, ovoid-shape, gourd-shape, or drop-shape as long as it is suitable to be worn between the labia. However, with the same shape as in the embodiment, it can be provided to easily fit for both the labia and the finger.

Each sheet of the interlabial pad 1 according to the embodiment is made of a flexible sheet so that freely and elastically deformed by the external pressure. Hence, the shape of the interlabial pad 1 may be deformed on the appearance such as being bent or twisted, however, the above-described shape is restored by removing such deformation from each sheet and stretching it. Specifically, there may be a case where the longitudinal cross sectional shape of the hollow part 5 in the lateral direction is deformed to be elliptic or circle along with the peripheral edge shape in the finger thickness direction at the time of finger insertion, or deformed to be in contact with the neighboring surface being bent towards the inside of the hollow part 5 before used. However, the substantial triangular is achieved by stretching each surface.

### [Material]

The material used for the surface side sheet 11 of the main sheet body 2 and the surface side sheet 61 of the sub-sheet body 6 are not specifically limited as long as it has the structure which permeates a liquid, such as a fabric, nonwoven fabric or perforated plastic sheet. In addition to a perforated film obtained by performing perforation, heat embossing, machine processing or the like on a thermoplastic film, a composite sheet of the perforated film and the nonwoven fabric, the materials shown below can be also used.

As the fabric and nonwoven fabric, examples of the natural fibers are cotton, silk, and hemp, examples of the regenerated fibers are regenerated cellulose fiber such as rayon fiber and acetate fiber, and examples of synthetic fibers are a single fiber and a composite fiber with a sheath-core structure or the like made of polyolefin fiber, polyacrylonitrile fiber, polyester fiber, polyamide fiber, polyvinyl alcohol fiber, polyurethane fiber, nylon and the like. Especially for the nonwoven fabrics, web forming can be performed either by dry method (carding, spun bonding, melt-blown, air-laid and the like) or wet method, or a plurality of the methods may be combined to be used. Examples of bonding methods are spun lacing using columnar water flow, thermal bonding, and needle punching.

Among the materials, considering the liquid mobility from the inner face of the labia, chemical stimulation by an activator, and adhesion with the inner wall of the labia, it is preferable to laminate rayon with 1.1 to 4.4 dtex fineness and 7 to 51 mm fiber length by 40 to 80 % of a total specific weight per unit area on the body surface side, and to laminate a mixture of rayon with 1.1 to 4.4 dtex fineness and 7 to 51 mm fiber length by 14 to 42 % of a total specific weight per unit area and PET with 1.1 to 4.4 dtex fineness and 7 to 51 mm fiber length by 6 to 18 % of a total specific weight per unit area on the clothing surface side. After laminating them so that the total specific weight per unit area of the two layers becomes 20 to 60 g/m², the fibers are entangled by water-flow interlacing treatment and then dried to prepare spun lace nonwoven fabric with the thickness of 0.13 to 0.50 mm. The spun lace nonwoven prepared as described is preferable. At this time, by mixing PET on the clothing side, bulkiness can be easily maintained even if the permeable sheet becomes wet. Therefore, adhesion between the inner wall of the labia can be maintained.

Examples of a perforated plastic sheet which can be used are an air sheet of thermoplastic resin such as polyethylene (PE), polypropylene (PP), and polyethylene terephthalate (PET), and a perforated foamed material. Also, it is preferable to use it by making it milky by mixing a filler made of titanium oxide, calcium carbonate and the like within the range of 0.5 to 10 weight % if necessary. A perforated film obtained by forming perforation, thermal embossing, machine processing or the like on a thermoplastic film may be used. Furthermore, a composite sheet of the perforated film and nonwoven fabric may be used.

The material used for the absorbent body 13 and the absorbent body 63 may be any material as long as it is capable of absorbing and holding a liquid (blood). However, it is preferable to use a single material or a mixture of the materials selected from the group comprising pulp, chemical pulp, rayon, acetate, cotton, particulate polymeric absorbent body, fiber polymeric absorbent body, and a composite fiber. The method by which the materials are formed to be the absorbent body is not limited, however, the method such as air-laid, melt-blown, spun lacing, or paper-making method is employed for an absorbent body to be formed into a sheet to be used. Also, cellulose foam, a continuous foam and the like of synthetic resin can be also used as the absorbent body. Furthermore, an absorbent body obtained by grinding and molding the above-described sheet and the foam can be used.

It is preferable for the absorbent body, although any material can be used as long as it is capable of absorbing and holding liquid (fluid), to be bulky, hard-to-be deformed, less chemically stimulant, and highly flexible to fit between the labia. Specifically, a nonwoven sheet in which, 50 to 150 g / m² of pulp selected from the range of the fiber length of 1 to 10 mm is laminated on the garment face side and, on the body face side, 150 to 250 g / m² of a mixture obtained by mixing 60 to 90 % of rayon with 1.1 to 4.4 dtex fineness and 20 to 51 mm fiber length with 40 to 10 % of natural cotton by this mixing ratio is laminated, which then to be formed into a sheet by dotted embossing to have 2 to 10 mm bulkiness, and more preferable to have 3 to 5 mm bulkiness. Thereby, liquid can be easily transmitted from the body face side to the garment face side resulting in the improvement of the absorbing and holding capacity. Furthermore, by providing a mesh spun lace nonwoven fabric of rayon with 1.1 to 4.4 dtex fineness and 25 to 51 mm fiber length by a specific weight per unit area of 15 to 40 g / m², the liquid transmitted from the body face side can be dispersed by the mesh spun lace to be induced to almost all over the region of the pulp layer. Therefore, more liquid can be effectively absorbed.

The material used for the back side sheet 12 of the main sheet body 2 and the back side sheet 62 of the sub - sheet body 6 are not specifically limited as long as it has a sheet - type structure such as a fabric, nonwoven fabric, or a plastic. However, examples of an impermeable material are an impermeable film mainly made of PE, PP or the like, a breathing resin film, and a material in which a breathing resin film is bonded to the back side of a nonwoven fabric such as a spun bond or spun lace on which water-repellent processing is performed. Considering the degree of softness by which the wear feeling is not influenced, for example, a film obtained by a specific weight per unit area of 15 to 30 g/m² mainly using LDPE (low density polyethylene) is used.

By preparing the back side sheet 62 of the above-described sub-sheet body 6 using an impermeable material, blood held in the absorbent body 63 can be prevented from leaking out. Also, by preparing it using a wet permeable material, stuffiness can be decreased when wearing. Thereby, uncomfort felt by the wearers can be decreased when wearing. It is more preferable to reduce the contact ratio to decrease the friction drag value by embossing the above-described film to provide convex-shaped projections in order to, when the pad is worn between the labia, decrease the risk of the interlabial pad from being fallen off from the labia due to the high friction caused by the contact between the impermeable sheets, or with a pad used together, an underwear or the like.

It is preferable to select the material used for the mini sheet piece 14 considering the strength of the material so that it is not damaged when a finger is inserted. It is possible to select with no limitation a single material or the laminated material from the group comprising a nonwoven sheet, an elastic dilation nonwoven fabric, a film, a foam film, an elastic dilation film, a foam sheet, a tissue paper, and the like. A specific example is a film of 15 to 30 µm thickness having an LDPE resin as the main component. Also, the mini sheet piece 14 can be prepared to have the tone of color, design, chroma which are different from those of the back side sheet 62 of the interlabial pad 1 by coloring or printing a design or the like in order for the wearer to be able to easily discriminate the mini sheet piece 14.

In order to effectively use the interlabial pad 1 according to the present invention, it is also effective to prepare the above-described mini sheet piece 14 to have a characteristic of stretching or elastic dilation in the lateral direction of the back side sheet 12 regardless of the finger size of the wearer.

In order for the mini sheet piece 14 to have a stretching characteristic, a stretching spun bond nonwoven fabric can be used in which the stress is 0.1 to 0.5 N/25 mm at the time of 5 % stretching when being stretched at a constant speed by a stretching speed of 100 m/minute with a grip interval of 100 mm.

Also, in order for the mini sheet piece 14 to have an elastic dilation characteristic, a fiber sheet or film sheet using thermoplastic elastomer resin may be used. Also, the elastic dilation material such as the thermoplastic elastomer resin or natural rubber may be used alone or may be combined with a non-elastic dilation material to be used.

The interlabial pad 1 of the present invention can be formed of a biodegradable material, a water dispersible material, a water soluble material, or any combination of these materials. Thereby, the interlabial pad 1 after being used is to be naturally decomposed as time goes by or actively. Therefore, it can be flushed down to a toilet so that discard of the used interlabial pad 1 can be performed easily and cleanly. In other words, the wearer, when discarding the interlabial pad 1, simply goes to a toilet and open the leg towards the toilet bowl to drop the interlabial pad 1 into the toilet bowl. Hence, there is no need for the wearer to be bothered going through a complicated action such as discarding the used product using hands. In addition, there is an advantage that trashes left in the toilet can be decreased.

Furthermore, by preparing the wrapping container for individually wrapping the interlabial pad 1 according to the present invention by a biodegradable material and / or a water soluble material and / or water dispersible material, the wrapping container can be also flushed down to the toilet. Thereby, the wearer can be freed from the trouble of discarding the wrapping container and trashes in the toilet can be further decreased at the same time.

In this Specification, "biodegradable" means that a substance is decomposed into gas such au carbon dioxide and methane, water, and biomass under an anaerobic or aerobic condition according to the natural process under the existence of fungi, bacteria, Actinomycetes and other microbes, and also means that the biodegradability of the synthetic material such as biodegradable rate and biodegradable degree equals to a material naturally generated such as fallen leaves or a synthetic polymer generally recognized having the same biodegradability under the same environment. "Water dispersible" has the same meaning as water degradable. It means a characteristic in which, while having no influence when used in a limited amount of moisture (blood), in a large amount of water or water current, the fabric is easily dispersed into small pieces at least to a degree where an ordinal toilet plumbing is not clogged. "Water soluble" is a characteristic in which, while having no influence when used in a limited amount of moisture (blood), the fabric is soluble in a large amount of water or water current.

The material is not specifically limited as long as it satisfies the above-mentioned conditions. However, the materials which can be used are shown below. First, a natural fiber and / or chemical fiber can be used for the fiber as a permeable material. Examples of the natural fiber are tissue, ground pulp, air laid pulp which is obtained by chemical-bonding a water soluble resin, and cotton. Examples of hydrophilic chemical fiber are rayon which is a regenerated cellulose, feeble rayon, and the like, and examples of synthetic fiber are the one obtained by performing hydrophilic processing on polyester, polypropylene, polyethylene, ethylene vinyl acetate copolymer and the like. Also, examples of synthetic biodegradable fiber is poly lactic acid, polybutylene succinate, and the like, and examples of a water soluble material are carboximethyl cellulose, polyvinyl alcohol, polyacrylonitrile and the like. Especially, it is preferable to use the natural fiber such as pulp or cotton or biodegradable fiber such as rayon or poly lactic acid. It is also possible to use one of these materials alone or by mixing the materials by a predetermined combination to form a web or nonwoven fabric. Web forming of the synthetic degradable fabric such as poly lactic acid or polybuthylene succinate may be performed using a dry method, wet method or the like by carding, spun bonding, melt blow and air laid, or may be performed by a method in which a plurality of the methods are combined.

Examples of boding are spun lace by columnar water flow, thermal bonding, needle punching, and chemical bonding. Example of method for forming a water dispersible fiber is a method of forming a water soluble paper in which a fiber is formed into a sheet by a hydrogen bonding of fibers, and a water soluble paper in which a fiber is formed into a sheet by entangling.

In order to keep an excellent water dispersiblity, it is preferable to have the fabric length within the range of 2 to 51 mm, and more preferable to have it within the range of 2 to 10 mm. Furthermore, if the water dispersiblity and strength enough for not-causing damage are considered, it is desirable to select the fineness of the fiber (thickness) within the range of 1.1 to 4.4 dtex.

Also, it is preferable to have a specific weight per unit area of 20 to 60 g/m². The break strength (the break strength when constant-stretching at grip interval of 100 mm and stretching speed at 100 mm/min) of the permeable material in both longitudinal and lateral direction are at least 800 mN/25 mm, and more preferable to be selected from the range of 1000 to 7000 mN/25 mm with the consideration of softness at the time of wearing.

A specific example of the permeable material is a wet forming spun lace nonwoven fabric prepared by mixing 5 to 10 mm of rayon fiber of 1.1 to 4.4 dtex and wood pulp at 90 : 10 to 70 : 30 weight ratio with a specific weight per unit area of 25 to 40 g/m² and the thickness of 0.2 to 0.5 mm. A plurality of pores may be provided on the permeable material. In this case, the pores may be formed to have a diameter within the range of 0.5 to 1.5 mm with the porous area ratio (rate of the porous area per unit area) within the range of 3 to 20 %.

A natural fiber and / or chemical fiber can be used for the absorbent body 13 and the absorbent body 63. Examples of the natural fiber are tissue, ground pulp, air laid pulp which is obtained by chemical bonding water soluble resin, cotton, and the like. Examples of hydrophilic chemical fiber are rayon which is a regenerated cellulose, feeble rayon, and the like, and examples of synthetic fiber are the one obtained by performing hydrophilic processing on polyester, polypropylene, polyethylene, ethylene vinyl acetate copolymer and the like. Also, examples of synthetic biodegradable fiber are poly lactic acid, polybutylene succinate, and the like, and examples of a water soluble material are carboximethyl cellulose, polyvinyl alcohol, and polyacrylonitrile. Especially, it is preferable to use the natural fiber such as pulp, cotton, or the like, or biodegradable fiber such as rayon, poly lactic acid, or the like. It is also possible to use one of these materials alone or by mixing the materials by a predetermined combination. It is also possible to form a high absorbent polymer such as sodium alginate, amylum, starch, carboxymethyl cellulose into grains or fibers and mixing it with the high absorbent polymer and the like at a predetermined combination.

An example of the absorbent body 13 as described is a material prepared by enclosing wood pulp by laminating it with a specific weight per unit area of 150 to 500 g/m² to tissue and prepare it to the thickness of 2 to 10 mm by a pressing device. By mixing 5 to 30 g/m² of an absorbent such as starch with the above - mentioned absorbent body, it is possible to also improve the absorbance and holding ability of blood.

Examples of the impermeable material with biodegradability and / or water solubility are cellulose derivative such as methyl cellulose, hydroxyethyl cellulose, carboxymethyl cellulose, etc., water soluble polymer such as polyvinyl alcohol, sodium alginate, sodium poly acrylate, polyacrylic ether, polyvinyl pyrrolidone, and a copolymer of isobutylene and maleic anhydride, poly lactic acid, polybutylene succinate, starch, dextrin, etc..

These materials may be used alone or mixed by a predetermined combination to be formed into a film sheet. Furthermore, repellent such as silicone may be applied or mixed thereto or it may be formed by a laminate processing performed on a nonwoven fabric.

A specific example of the sheet is a film obtained by preparing polyvinyl alcohol with a specific weight per unit area of 20 to 50 g/m² and to which 0.5 to 5 µm silicone or fluorine is applied at least on either side and, more preferably, on both sides.

Examples of the preferable material for the mini sheet piece 14 are polyvinyl alcohol film, and a laminated material of polyvinyl alcohol, tissue, etc.

As the applicable bonding, bonding by polyvinyl alcohol with water solubility or water dilatation characteristic, heat sealing, hydrogen bonding, etc. may be used alone or combined to be used as a bonding method.

Specific examples of the wrapping container made of a water soluble material or water dispersible material are a compound material obtained by laminating tissue with a specific weight per unit area of 15 to 40 g / m² and polyvinyl alcohol with a specific weight per unit area of 20 to 50 g / m² and applying silicone of 0.5 to 1 µm on the polyvinyl alcohol side, a spun bond nonwoven fabric, etc. prepared with a specific weight per unit area of 15 to 40 g/m² mainly using poly lactic acid fiber.

### [Size]

The length of the main sheet body 2 in the lateral direction on the appearance is preferable to be 10 to 60 mm, and more preferable to be 20 to 40 mm. When the length in the lateral direction is longer than 60 mm, the area which is not inserted between the labia is rubbed against the femoral region or the like of the wearer and the friction generated thereby exceeds the holding strength between the both labia so that the interlabial pad may fall off. Also, when the length in the lateral direction is shorter than 10 mm, the area which can be inserted between the labia becomes small thereby reducing the contact area with the inner face of the labia. Thereby, there generates a risk of the interlabial pad being fallen off.

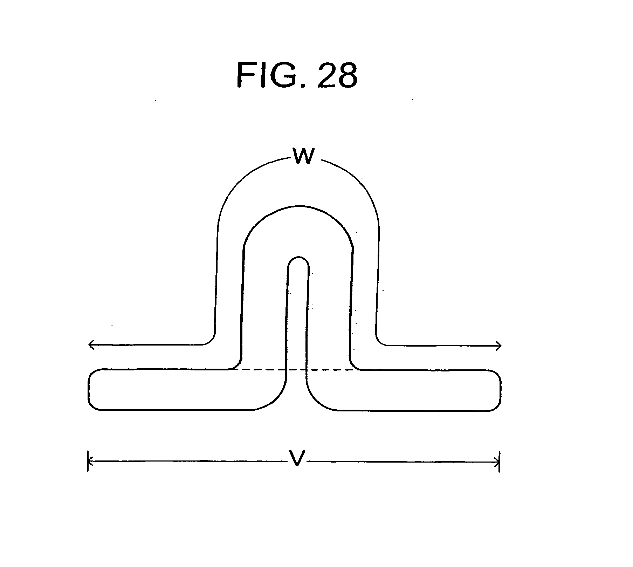

The above-described "appearance" means the distance between two points with the shortest length (V in Fig. 28). This is to carefully define the length since, in the manufacturing step, there may be a case where the length between the two points in a concave and convex shape is taken as the actual length (W in Fig. 28), that is, the distance between the two points in the state in which the concave and convex shape are unfolded to be flat.

On the other hand, the length of the main sheet body 2 in the longitudinal direction is preferable to be 50 to 150 mm, and more preferable to be 80 to 120 mm. When the length in the longitudinal direction is longer than 150 mm, friction generated by the substantial flat area 4 which is not inserted between the labia being rubbed against the underwear or a sanitary napkin exceeds the holding strength of the labia itself so that the interlabial pad 1 may fall off. Also, when the length in the longitudinal direction is shorter than 50 mm, the range of the area of main sheet body 2 which can be inserted between the labia becomes small thereby reducing the contact area between the labia and the main sheet body 2. Thereby, there generates a risk of the interlabial pad 1 being fallen off.

The length of the sub-sheet body 6 in the lateral direction on the appearance is preferable to be 10 to 60 mm, and more preferable to be 30 to 40 mm. When the length in the lateral direction is longer than 60 mm, the end of the substantial flat area 4 is rubbed against the femoral region of the wearer thereby generating friction. The generated friction exceeds the holding strength of the both labia so that the interlabial pad 1 may fall off. Also, when the length of the sub-sheet body 6 in the lateral direction is shorter than 10 mm, it becomes shorter on the appearance than the maximum value of the length of the unbonded part, which is not inserted between the labia, in the main sheet body 2 in the lateral direction. As a result, the range of the substantial flat area 4 in the lateral direction functioning to absorb blood which cannot be completely absorbed in the long convex area 3 of the main sheet body 2 becomes insufficient for covering the pudenda.

The above-described "appearance" means the distance between two points with the shortest length. This is to carefully define the length since, in the manufacturing step, there may be a case where the "distance" between the two points in a concave and convex shape (that is, the distance between the two points in the state in which the concave and convex shape are unfolded to be flat) is taken as the actual "length".

On the other hand, the length of the sub - sheet body 6 in the longitudinal direction is preferable to be 60 to 160 mm, and more preferable to be 90 to 130 mm. When the length of the sub - sheet body 6 in the longitudinal direction is longer than 160 mm, friction may be easily generated since the sub - sheet body 6 keeping a plan shape is rubbed against the napkin or the underwear, and the generated friction exceeds the holding strength of the labia itself so that the interlabial pad 1 may easily fall off. Also, when the length of the sub-sheet body 6 in the longitudinal direction is shorter than 60 mm, it becomes shorter than the length of the main sheet body 2 in the longitudinal direction. Therefore, it becomes difficult to absorb blood leaked from the main sheet body 2 in the longitudinal direction so that blood outflow from the longitudinal direction likely to occur.

By providing each sheet body in the length within the range as described, the interlabial pad 1 having an excellent prevention effect of blood leak with an excellent wear feeling can be achieved.

### [Finger Insertion Opening]

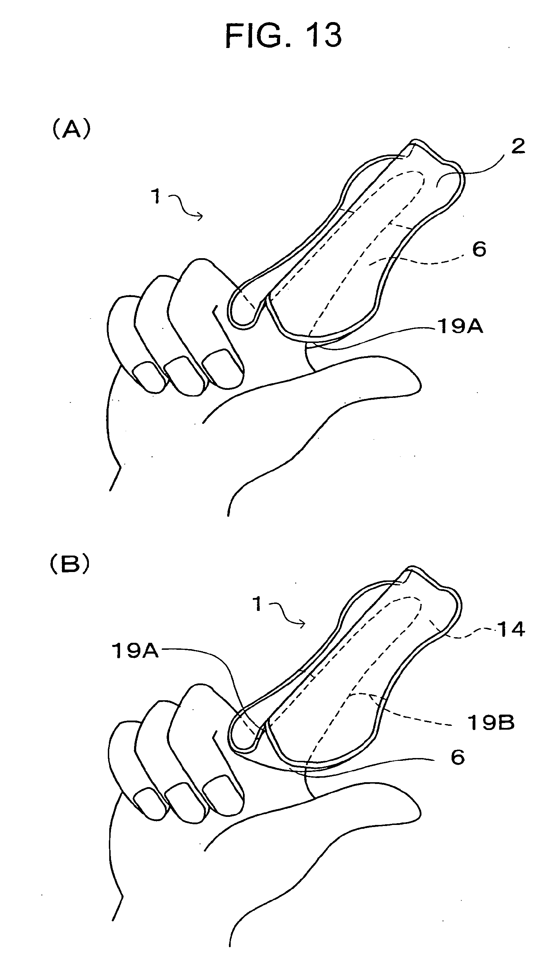

Next, the finger insertion opening 19A, the hollow part 5 continued therefrom, the finger insertion opening 19B and the hollow part 7 continued therefrom provided in the interlabial pad 1 according to the embodiment will be described. Fig. 11 is an explanatory illustration for describing the manner in which the forefinger is put in and out from the first finger insertion opening 19 A to the hollow part 5 when using the interlabial pad 1 according to the embodiment. Fig. 12 is an explanatory illustration for describing the manner in which the forefinger is put in and out from the second finger insertion opening 19 B to the hollow part 7 when using the interlabial pad 1 according to the embodiment. Fig. 13 is an explanatory illustration for describing that the first finger insertion opening 19A and the second finger insertion opening 19B face the same direction.

In the interlabial pad 1 according to the embodiment, the first finger insertion opening 19A is formed inside the long convex area 3 provided by bending the main sheet body 2, and the second finger insertion opening 19B is formed inside between the mini sheet piece 14, which is attached on the opposite side surface to the sub-sheet body 6, and the sub-sheet body 6. Thereby, the wearer can select either the first finger insertion opening 19A or the second finger insertion opening 19B in accordance with her labia depth. In other words, if the wearer has shallow labia depth, the finger can be inserted from the first finger insertion opening 19A to the hollow part 5 continued therefrom. On the contrary, if the wearer has deep labia depth, the finger can be inserted from the second finger insertion opening 19B to the hollow part 7 continued therefrom.

Specifically, the wearer with shallow labia depth, as shown in Fig. 11, inserts the finger from the first finger insertion opening 19A to the hollow part 5 having its fingerprint side face being in contact with the opposite side surface to body 12a of the back side sheet 12 in the main sheet body 2. In this case, the hollow part 5 becomes smaller in a substantially continuous manner. Therefore, the finger is to be inserted from the sleeve portion 5a with a large longitudinal cross sectional area to the sleeve portion 5b with small area. Also, the wearer with deep labia depth, as shown in Fig. 12, inserts the finger from the second finger insertion opening 19B to the hollow part 7 having its fingerprint side face being in contact with the opposite side surface to body 62a of the back side sheet 62 in the sub - sheet body 6.

As described, according to the present invention, when wearing the interlabial pad 1 between the labia, the ostium vagina with a concaved shape can be detected by the finger cushion of finger with a keen sense through the main sheet body 2 or both of the main sheet body 2 and the sub - sheet body 6. Thereby, the interlabial pad 1 can be led so that the long convex area 3 is inserted between the labia.

When wearing the interlabial pad 1 according to the embodiment, first, the forefinger or middle finger is inserted to the finger insertion opening to hold the product thereby. However, there are two finger insertion openings so that the wearer selects either one in accordance with her own labia depth. In the embodiment, the finger insertion opening 19A and the finger insertion opening 19B are positioned to face in the same direction so that there is no need for the wearer to turn over the whole body of the interlabial pad 1 when selecting either the finger insertion opening 19A or the finger insertion opening 19B. When the finger is inserted to the finger insertion opening 19A, as shown in Fig. 13(A), the finger is to be inserted between the main sheet body 2 and the sub - sheet body 6 and, when the finger is inserted to the finger insertion opening 19B, the finger is to be inserted between the sub-sheet body 6 and the mini sheet piece 14. Thereby, the interlabial pad 1 is fixed to the fingertip. Incidentally, when wrapping the interlabial pad 1 according to the present invention individually, by positioning the finger insertion opening 19A and the finger insertion opening 19B near the opening section, wearers with any types of the labia depth can easily insert their fingers.

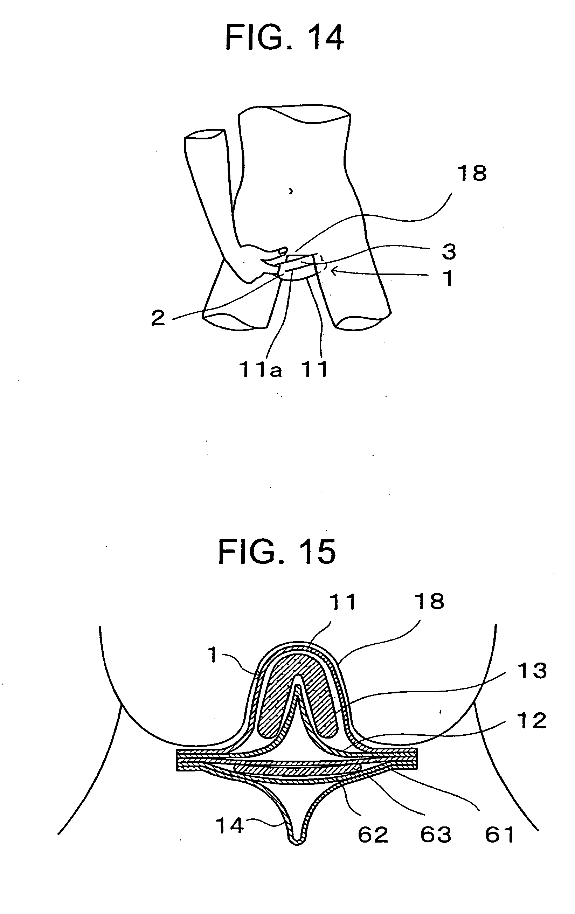

As described, by inserting the finger to the finger insertion opening 19A or 19B, the fingerprint side surface of a finger from the first joint up, where many receptors exist, comes to be in contact with the opposite side surface to the body 12a of the back side sheet 12. Therefore, when leading the interlabial pad 1 to the labia, as shown in Fig. 14, the opposite side surface to the body 11 a of the surface side sheet 11 in the main sheet body 2 is provided to be in contact with labia 18 and, while detecting the concave and convex of the labia 18 by the fingertip with a keen sense, the long convex area 3 formed on the body side surface of the interlabial pad 1 can be surely lead deep into between the labia 18 which is hard to be viewed.

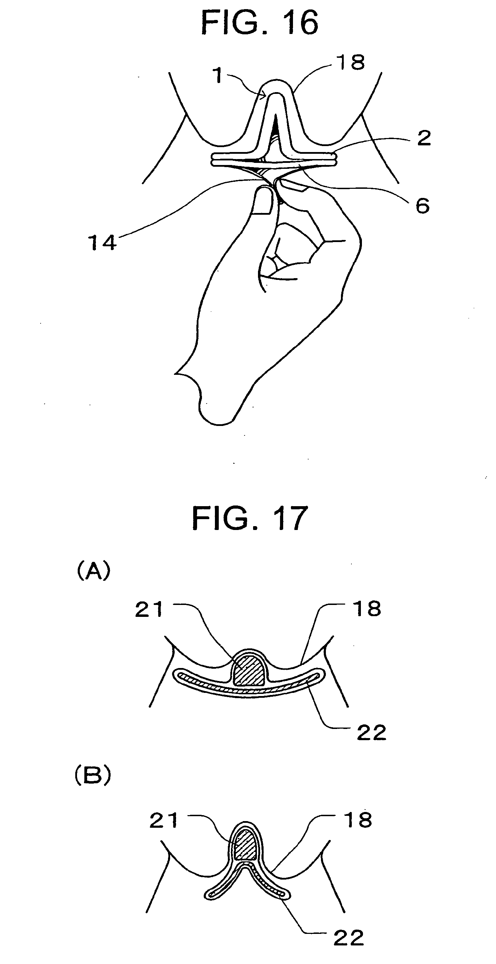

Inside of the mini sheet piece 14 forming the hollow part 7 which is the gap in the second finger insertion opening is not pasted to the back side sheet 62. Therefore, when the finger is pulled out from the finger insertion opening 19A after wearing the interlabial pad 1, as shown in Fig. 15, the mini sheet piece 14 is loosened in the opposite direction to the body side. Hence, when removing the used interlabial pad 1, the loosened mini sheet piece 14 can be pulled out as shown in Fig. 16. Furthermore, by providing the mini sheet piece 14 using an impermeable or wet permeable material, the interlabial pad 1 can be removed without contaminating the finger even if the wearer grabs the mini sheet piece 14.

By providing microscopic concaves and convexes in the area in the to be in contact with the finger cushion in the back side sheet 12 of the main sheet body 2 and the sub - sheet body 6, the area can be reduced where the finger cushion side of the fingertip comes to be in contact with the opposite side surface to the body 12a of the back side sheet 12 and the opposite side surface to the body 62a of the back side sheet 62. As a result, it makes possible to suppress friction and stuck generated between the fingertip and the interlabial pad 1. In this case, there is no chance for the interlabial pad 1 to be fitted in the position which is not the intention of the wearer, which may otherwise occur due to the influence of the state of the fingertip of the wearer, such as the wet environment. Also, the finger can be smoothly pulled out after wearing so that the position shift after wearing can be prevented.

### [Wearing State]

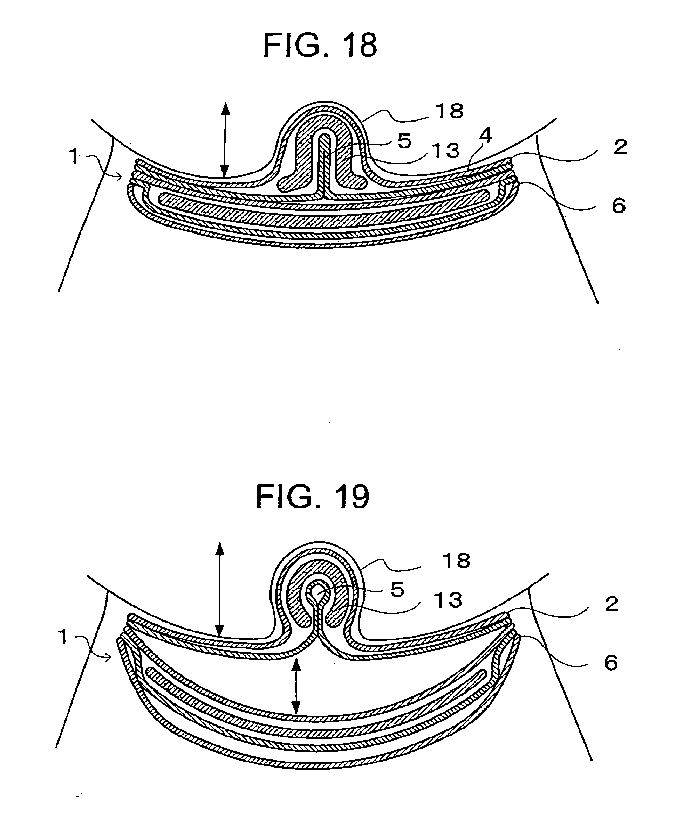

Next, the wearing state of the interlabial pad 1 according to the embodiment will be described. Fig. 17 is a cross section showing the wearing state of an interlabial pad with a ready - made projection 21. Fig. 18 and Fig. 19 are cross sections showing the wearing state of the interlabial pad according to the embodiment.

When wearing the interlabial pad 1 with the ready-made projection 21, if the height of the projection 21 and the depth of the labia 18 are not consistent with each other, there tends to be a gap generated between the labia 18 and the interlabial pad 1, which causes a bad wear feeling. In other words, as shown in Fig. 17(A), when the depth of the labia 18 is shorter than the height of the projection 21, the bottom portion of the projection 21 which is not inserted between the labia 18 separates the labia 18 and the part other than the projection 21, which is a flat part 22, thereby generating a gap therebetween. On the other hand, as shown in Fig. 17(B), when the depth of the labia 18 is longer than the height of the projection 21, not only the projection 21 but also the whole thickness of the flat area 22 positioned in the periphery of the projection 21 is inserted in between the labia 18 so that the wearer may feel a strong foreign feeling.

In this respect, in the interlabial pad 1 according to the present invention, when the height of the long convex area 3 is shorter than the depth of the labia 18, a finger is inserted from the first finger insertion opening 19A to fix the interlabial pad 1 to the fingertip and detect the ostium vagina by having the fingerprint surface of the finger being in contact with the opposite side surface to the body 12a of the back surface side sheet 12 so as to fit the interlabial pad 1. Thereby, the long convex area 3 is inserted between the labia 18. After wearing the interlabial pad 1, as shown in Fig. 18, only a part of the long convex area 3 which can be inserted in between the labia 18 is inserted between the labia 18. In other words, if the height of the long convex area 3 is within the same range as that of the depth of the labia 18, the long convex area 3 is inserted in between the labia 18 within the range. If the height is out of the range, the both side faces of the substantial triangular can be collapsed towards inside and deformed to fill the hollow part 5. As described, according to the present invention, there is no gap generated between the labia 18 and the interlabial pad 1. Therefore, leak of blood can be decreased and a preferable wear feeling can be achieved.

When the height of the long convex area 3 is longer than the depth of the labia 18a wearer insert the finger from the second finger insertion opening 19B to fix the interlabial pad 1 to the fingertip. The main sheet body 2 and the sub-sheet body 6 can be separated in the outer edge to the inner side so that after wearing, as shown in Fig. 19, the main sheet body 2 near the long convex area 3 can be also inserted between the labia 18. As described, only the main sheet body 2 is inserted between the labia 18 but the sub-sheet body 6. Therefore, compared to an interlabial pad with a single structure in which the whole thickness of the interlabial pad 1 is inserted in between the labia 18, a foreign feeling felt by the wearer can be remarkably decreased.

As described, according to the present invention, a gap generated between the pudenda of the wearer and the pad is decreased and there is no influence on the wear feeling. When the long convex area 3 is inserted between the labia 18, the substantial flat area 4 is positioned to cover the pudenda from the outside so that the adhesion between the interlabial pad 1 and the inner thigh of the wearer can be improved.

### [Application of Adhesive]

In order to decrease a risk of fall-off of the interlabial pad 1, by applying an adhesive beforehand on a part of the surface side sheet 11 of the main sheet body 2 and / or the surface side sheet 61 of the sub-sheet body 6, the fixing and the adhesion of the interlabial pad 1 between the labia can be improved. Thereby, generation of a gap between the body and the interlabial pad due to the body action of the wearer can be prevented.

As the adhesive agent as described, a gel adhesive, etc. made of water-soluble polymer, a crosslinking agent, a flexibilizer and moisture can be used. Examples of the water soluble polymer used herein is gelatin, polyacrylic acid sodium, polyvinyl alcohol, and carboxymethyl cellulose, etc. Examples of the crosslinking agent are water soluble metallic salt such as calcium chloride and magnesium salfate and examples of the flexibilizer are glycelol, wax, paraffin, etc.

As other adhesive agent, so-called a pressure sensitive hot melt can be also used. Incidentally, the pressure sensitive hot melt is mainly formed of synthetic rubber resin such as styrene-isoprene-styrene block copolymer (SIS), styrene-butadiene-styrene block copolymer (SBS), styrene-ethylene-butadiene-styrene block copolymer (SEBS), and styrene-ethylene-propylene-styrene block copolymer (SEPS). The pressure sensitive hot melt adhesive can be obtained by fused-mixing adhesion adder such as terpene resin or rosin resin and a flexibilizer such as wax, etc. to the pressure sensitive hot melt. Also, silicone adhesive agent can be used as other adhesive. An example of the silicone adhesive is a mixture obtained by mixing a crosslinking agent such as metallic salt of platinum, molybdenum, or antimony and a flexibilizer such as ester wax, glycerin, or machine oil, etc.

If the application stability is taken into consideration, the pressure sensitive hot melt is preferable. More specifically, it is an adhesive agent prepared by fused - mixing 15 to 25 weight % of SEBS, 15 to 35 weight % of flexibilizer, and 40 to 70 weight % of adhesive adder. Also, according to circumstances, it is possible to add antioxidant, antifluorescent or the like within the range of 0.1 to 1.0 weight % to the pressure sensitive hot melt.

It is preferable to cover the part where the adhesive is applied with a sheet which is obtained by coating silicon resin on a tissue paper, which is a generally obtainable separate paper, or a sheet obtained by coating silicon resin on a film. Thereby, damages or separation of the adhesive part can be prevented while being stored.

Examples of the manner in which the adhesive is applied are in the form of whole surface application or in dots, mesh, or lines. The application position of the adhesive agent is not specifically limited as long as it enables fixing of the pad to the body. However, specifically considering the existence of the hair-grown part in the area in front of the labia, it is preferable to apply the adhesive near both end sides of the inter labia pad 1 in lines with about 1 to 5 mm width.

An example of valuation method of the adhesive strength will be described in detail. The valuation method is to measure the separation force (Fig. 20) and the shearing force of the adhesive (Fig. 21). A constant speed expansion tensile tester and a stainless plate of 80 mm x 50 mm is required as the instruments used therein. As a preparation for the evaluation test, a test piece of a polyethylene film 36 in which an adhesive 37 is applied within the range of 25 mm in width and 50 mm in length is left for 30 minutes at a room temperature of 20 °C beforehand. Subsequently, the polyethylene film 36 is put lightly over a stainless plate 35 with the width being overlapped and the adhesive 37 being in contact with the stainless plate 35, and a 2 kg-roller is applied one way. Then, it is left for 30 minutes at a room temperature of 20 °C.

The test sheet obtained as described is used, and the test condition is provided to be 70 mm chuck interval (grip interval) and 100 mm/min testing speed. In the separation force test of the adhesive, it is separated in the pulling direction B in Fig. 20 and, in the shearing force test of the adhesive, it is pulled in the pulling direction C in Fig. 21.

In the case where the forces are measured by the measurement method described above, considering the burden imposed on the skin of the wearer, it is preferable that the measurement value of the separation force to be 100 to 2000 mN/25 mm and that of the shearing force to be 2900 to 15000 mN/25 mm.

### [Individual wrapping]

When individually wrapping the interlabial pad 1 according to the present invention, it is preferable to prepare the pad so that a finger can be inserted to the finger insertion opening 19A or finger insertion opening 19B right after opening the wrapping container. For example, the pad may be wrapped anisotropic to the wrapping container so that the opening direction and the fingertip insertion direction become the same, or the mini sheet piece 14 for finger insertion can be positioned to be near the opening section of the wrapping container.

It is also preferable to wrap the interlabial pad 1 by folding it in such manner that the finger insertion opening 19A and the finger insertion opening 19B are naturally opened when opening the wrapping container. Thereby, the wearer can easily recognize the position of finger insertion. As a result, the interlabial pad 1 can be fitted more quickly and easily.

When folding the interlabial pad 1, for example, as shown in Fig. 22A, the wearer with shallower labia depth can easily insert the finger by folding it with the finger insertion opening 19A being the top face and, as shown in Fig. 22B, the wearer with the deeper labia depth can easily insert the finger by folding it with the finger insertion opening 19B being the top face.

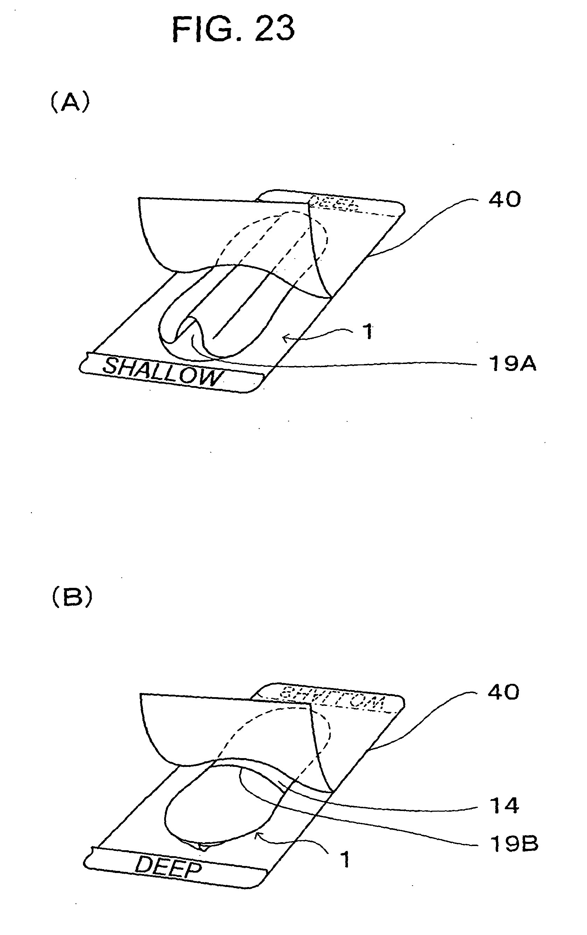

Furthermore, by specifying the breaking direction of the opening section in accordance with the labia depth through providing a character or the like in the wrapping container 40, it can be also made easier for the wearer to insert the finger to the finger insertion opening suitable for her own labia depth. For example, the interlabial pad 1 is wrapped in the wrapping container 40 with the finger insertion opening 19A positioned near the opening section where a character meaning "shallow" is put, and the finger insertion opening 19B positioned near the opening section where a character meaning "deep" is put. Thereby, the wearer with the shallow labia depth can insert the finger easily from the finger insertion 19A by opening the wrapping container 40 from the opening section where a character meaning "shallow" is put as shown in Fig. 23A. Then, the wearer with the deep labia depth can insert the finger easily from the finger insertion 19B by opening the wrapping container from the opening section where a character meaning "deep" is put as shown in Fig. 23B.

### [Other Applicable Embodiment of the Interlabial pad]

The interlabial pad 1 according to the embodiment, as shown in Fig. 24, can be used together with an ordinal sanitary napkin 30. As for the wearing method, the interlabial pad 1 is fitted in between the labia and the sanitary napkin 30 is fitted to the underwear. By using it together with a sanitary napkin as described, the interlabial pad 1 of the present invention can be effectively used even on a occasion expecting a large quantity of blood.

### Industrial Applicability

According to the present invention as described, a finger can be inserted to a finger insertion opening provided in an interlabial pad having a long convex area on the body side face. Thereby, the interlabial pad can be fixed and held by the fingertip so that the interlabial pad can be fitted in the appropriate position even in between labia where it is hard to be viewed.

Also, the above-described finger insertion opening is provided in two areas so that the wearer can select either one according to her own labia depth. In addition, the above-described long convex area are flexibly deformed in accordance with the labia depth of the wearer thereby enabling a close wearing of the interlabial pad in between the labia regardless of the labia depth of the wearer. As a result, leak of blood can be drastically decreased.

## Claims

1. An interlabial pad (1) for inserting between female labia, comprising:
a main sheet body (2) composed of a water permeable surface sheet (11) facing a body side and a water permeable back side sheet (12) facing an opposite side of the body side, the main sheet body (2) containing an absorbent body (13) for absorbing body liquid, the absorbent body (13) being enclosed by and between the surface sheet (11) and the back side sheet (12), the surface sheet (11) and the back side sheet (12) being bonded to each other; and
a sub-sheet body (6) composed of a water permeable surface sheet (61) positioned at the body side and a water non-permeable back side sheet (62) facing a clothes side, the sub-sheet body (6) containing an absorbent body (63) for absorbing liquid, the absorbent body (63) being enclosed by and between the surface sheet (61) and the back side sheet (62), the surface sheet (61) and the back side sheet (62) being bonded to each other;
wherein said main sheet body (2) comprises a long convex area (3) formed along a longitudinal direction of the surface side sheet (11) so that a centre area of the surface sheet (11) in a lateral direction is formed convex towards the body side;
**characterised in that** the long convex area (3) has a hollow part (5) formed between the main sheet body (2) and the sub-sheet body (6) with sleeve portions (5a, 5b) located at both ends of the hollow part (5) in the longitudinal direction, the main sheet body (2) and the sub-sheet body (6) are bonded at each longitudinal side edge and at least one of the sleeve portions (5a, 5b) is unbonded in the lateral direction, wherein the unbonded sleeve portion comprises a sleeve opening that forms a finger insertion opening (19A) for insertion of a finger.

2. An interlabial pad (1) according to claim 1 wherein the main sheet body (2) and the sub-sheet body (6) are bonded at each adjacent peripheral edge (15, 65) and are not bonded inside the peripheral edges (15, 65).

3. An interlabial pad (1) according to claim 1 or 2 wherein the main sheet body (2) comprises a plurality of main sheet bodies formed in the longitudinal direction that are each bonded together at each longitudinal side edge and are each unbonded at at least one end in the lateral direction so as to form finger insertion openings in which a finger can be inserted.

4. An interlabial pad (1) according to any of claims 1 to 3 comprising a mini sheet piece (14) fixed on the back side sheet (62) of the sub-sheet body (6) at the clothes side, the mini sheet piece (14) forming a finger insertion opening (19B) between the back side sheet (62) and the mini sheet piece (14).

5. An interlabial pad (1) according to any of claims 1 to 4 wherein the long convex area (3) is composed of a folded portion formed by folding the main sheet body (2).

6. An interlabial pad (1) according to any of claims 1 to 5 wherein the long convex area (3) is formed with a shorter top length than a bottom length in the longitudinal direction and wherein the sleeve opening is formed with both side edges sloped from a bottom to a top.

7. An interlabial pad (1) according to any of claims 1 to 6 wherein the long convex area (3) has a triangular longitudinal cross section.

8. An interlabial pad (1) according to any of claims 1 to 7, wherein a longitudinal cross sectional area of the long convex area in the lateral direction is at least 1 cm².

9. An interlabial pad (1) as claimed in any of claims 1 to 8 wherein a longitudinal cross sectional area of the long convex area (3) becomes continuously decreased as the area is taken from one end to the other end along the longitudinal direction.

10. An interlabial pad (1) according to any of claims 1 to 9 wherein the interlabial pad (1) is a sanitary-napkin-coexisting-interlabial pad that is used together with a sanitary napkin.

11. An interlabial pad (1) according to any of claims 1 to 10 wherein the interlabial pad (1) comprises an incontinent-interlabial pad for incontinence.

12. An Interlabial pad (1) according to any of claims 1 to 10 wherein the interlabial pad comprises a vaginal-discharge interlabial pad for absorbing vaginal discharge.

## Patentansprüche

1. Ein interlabiales Kissen (1) zum Einführen zwischen weibliche Labia, das Folgendes beinhaltet:
einen Hauptschichtkörper (2), der aus einer wasserdurchlässigen Oberflächenschicht (11), die einer Körperseite zugewandt ist, und einer wasserdurchlässigen Rückseitenschicht (12), die einer der Körperseite entgegengesetzten Seite zugewandt ist, zusammengesetzt ist,
wobei der Hauptschichtkörper (2) einen absorbierenden Körper (13) zum Absorbieren von Körperflüssigkeit enthält,
wobei der absorbierende Körper (13) von und zwischen der Oberflächenschicht (11) und der Rückseitenschicht (12) eingeschlossen ist, wobei die Oberflächenschicht (11) und die Rückseitenschicht (12) miteinander verbunden sind; und
einen Unterschichtkörper (6), der aus einer wasserdurchlässigen Oberflächenschicht (61), die an der Körperseite positioniert ist, und einer wasserundurchlässigen Rückseitenschicht (62), die einer Kleidungsseite zugewandt ist, zusammengesetzt ist, wobei der Unterschichtkörper (6) einen absorbierenden Körper (63) zum Absorbieren von Flüssigkeit enthält, wobei der absorbierende Körper (63) von und zwischen der Oberflächenschicht (61) und der Rückseitenschicht (62) eingeschlossen ist, wobei die Oberflächenschicht (61) und die Rückseitenschicht (62) miteinander verbunden sind;
wobei der Hauptschichtkörper (2) einen langen konvexen Bereich (3) beinhaltet, der entlang einer Längsrichtung der Oberflächenseitenschicht (11) gebildet ist, so dass ein in einer lateralen Richtung mittlerer Bereich der Oberflächenschicht (11) zur Körperseite hin konvex gebildet ist;
**dadurch gekennzeichnet, dass** der lange konvexe Bereich (3) einen hohlen Teil (5) aufweist, der zwischen dem Hauptschichtkörper (2) und dem Unterschichtkörper (6) gebildet ist, wobei Manschettenabschnitte (5a, 5b) in der Längsrichtung an beiden Enden des hohlen Teils (5) befindlich sind, dass der Hauptschichtkörper (2) und der Unterschichtkörper (6) an jedem Längsseitenrand verbunden sind und dass mindestens einer der Manschettenabschnitte (5a, 5b) in der lateralen Richtung unverbunden ist, wobei der unverbundene Manschettenabschnitt eine Manschettenöffnung beinhaltet, die eine Fingereinführungsöffnung (19A) zum Einführen eines Fingers bildet.

2. Interlabiales Kissen (1) gemäß Anspruch 1, wobei der Hauptschichtkörper (2) und der Unterschichtkörper (6) an jedem benachbarten peripheren Rand (15, 65) verbunden sind und innerhalb der peripheren Ränder (15, 65) nicht verbunden sind.

3. Interlabiales Kissen (1) gemäß Anspruch 1 oder 2, wobei der Hauptschichtkörper (2) eine Vielzahl von in der Längsrichtung gebildeten Hauptschichtkörpern beinhaltet, die jeweils an jedem Längsseitenrand miteinander verbunden sind und die jeweils an mindestens einem Ende in der lateralen Richtung unverbunden sind, um Fingereinführungsöffnungen zu bilden, in die ein Finger eingeführt werden kann.

4. Interlabiales Kissen (1) gemäß einem der Ansprüche 1 bis 3, das ein Minischichtstück (14) beinhaltet, welches an der Rückseitenschicht (62) des Unterschichtkörpers (6) an der Kleidungsseite fixiert ist, wobei das Minischichtstück (14) zwischen der Rückseitenschicht (62) und dem Minischichtstück (14) eine Fingereinführungsöffnung (19B) bildet.

5. Interlabiales Kissen (1) gemäß einem der Ansprüche 1 bis 4,
wobei der lange konvexe Bereich (3) aus einem gefalteten Abschnitt zusammengesetzt ist, der durch das Falten des Hauptschichtkörpers (2) gebildet wird.

6. Interlabiales Kissen (1) gemäß einem der Ansprüche 1 bis 5,
wobei der lange konvexe Bereich (3) in der Längsrichtung mit einer kürzeren Oberseitenlänge als Unterseitenlänge gebildet ist, und wobei die Manschettenöffnung so gebildet ist, dass beide Seitenränder von einer Unterseite zu einer Oberseite abgeschrägt sind.

7. Interlabiales Kissen (1) gemäß einem der Ansprüche 1 bis 6,
wobei der lange konvexe Bereich (3) einen dreieckigen Längsquerschnitt aufweist.

8. Interlabiales Kissen (1) gemäß einem der Ansprüche 1 bis 7,
wobei eine Längsquerschnittsfläche des langen konvexen Bereichs in der lateralen Richtung mindestens 1 cm² beträgt.

9. Interlabiales Kissen (1) gemäß einem der Ansprüche 1 bis 8,
wobei eine Längsquerschnittsfläche des langen konvexen Bereichs (3) kontinuierlich abnimmt, wenn die Fläche entlang der Längsrichtung von einem Ende zu dem anderen Ende genommen wird.

10. Interlabiales Kissen (1) gemäß einem der Ansprüche 1 bis 9,
wobei das interlabiale Kissen (1) ein mit Damenbinden vereinbares interlabiales Kissen ist, das zusammen mit einer Damenbinde verwendet wird.

11. Interlabiales Kissen (1) gemäß einem der Ansprüche 1 bis 10,
wobei das interlabiale Kissen (1) ein interlabiales Inkontinenzkissen für Inkontinenz beinhaltet.

12. Interlabiales Kissen (1) gemäß einem der Ansprüche 1 bis 10,
wobei das interlabiale Kissen ein interlabiales Scheidenausflusskissen zum Absorbieren von Scheidenausfluss beinhaltet.

## Revendications

1. Une protection interlabiale (1) pour insertion entre les lèvres génitales de la femme, comprenant :
un corps formant feuillet principal (2) composé d'un feuillet de surface perméable à l'eau (11) faisant face à un côté corps et un feuillet côté dos perméable à l'eau (12) faisant face à un côté opposé du côté corps, le corps formant feuillet principal (2) contenant un corps absorbant (13) pour absorber du liquide corporel, le corps absorbant (13) étant enfermé par et entre le feuillet de surface (11) et le feuillet côté dos (12), le feuillet de surface (11) et le feuillet côté dos (12) étant collés l'un à l'autre ; et
un corps formant sous-feuillet (6) composé d'un feuillet de surface perméable à l'eau (61) positionné au niveau du côté corps et un feuillet côté dos non perméable à l'eau (62) faisant face à un côté vêtements, le corps formant sous-feuillet (6) contenant un corps absorbant (63) pour absorber du liquide, le corps absorbant (63) étant enfermé par et entre le feuillet de surface (61) et le feuillet côté dos (62), le feuillet de surface (61) et le feuillet côté dos (62) étant collés l'un à l'autre ;
où ledit corps formant feuillet principal (2) comprend une longue zone convexe (3) formée le long d'une direction longitudinale du feuillet côté surface (11) de sorte qu'une zone centrale du feuillet de surface (11) dans une direction latérale soit de formation convexe en direction du côté corps ;
**caractérisée en ce que** la longue zone convexe (3) a une partie creuse (5) formée entre le corps formant feuillet principal (2) et le corps formant sous-feuillet (6), des portions de manchon (5a, 5b) étant situées au niveau des deux extrémités de la partie creuse (5) dans la direction longitudinale, le corps formant feuillet principal (2) et le corps formant sous-feuillet (6) sont collés au niveau de chaque bord de côté longitudinal et au moins l'une des portions de manchon (5a, 5b) n'est pas collée dans la direction latérale, où la portion de manchon non collée comprend une ouverture de manchon qui forme une ouverture d'insertion de doigt (19A) destinée à l'insertion d'un doigt.

2. Une protection interlabiale (1) selon la revendication 1 où le corps formant feuillet principal (2) et le corps formant sous-feuillet (6) sont collés au niveau de chaque bord périphérique adjacent (15, 65) et ne sont pas collés à l'intérieur des bords périphériques (15, 65).

3. Une protection interlabiale (1) selon la revendication 1 ou la revendication 2 où le corps formant feuillet principal (2) comprend une pluralité de corps formant feuillet principal formés dans la direction longitudinale qui sont chacun collés ensemble au niveau de chaque bord de côté longitudinal et dont aucun n'est collé au niveau d'une extrémité au moins dans la direction latérale de manière à former des ouvertures d'insertion de doigt dans lesquelles il est possible d'insérer un doigt.

4. Une protection interlabiale (1) selon n'importe lesquelles des revendications 1 à 3 comprenant un morceau formant mini-feuillet (14) fixé sur le feuillet côté dos (62) du corps formant sous-feuillet (6) au niveau du côté vêtements, le morceau formant mini-feuillet (14) formant une ouverture d'insertion de doigt (19B) entre le feuillet côté dos (62) et le morceau formant mini-feuillet (14).

5. Une protection interlabiale (1) selon n'importe lesquelles des revendications 1 à 4 où la longue zone convexe (3) est composée d'une portion pliée formée en pliant le corps formant feuillet principal (2).

6. Une protection interlabiale (1) selon n'importe lesquelles des revendications 1 à 5 où la longue zone convexe (3) est formée de façon à avoir une longueur en haut plus courte qu'une longueur en bas dans la direction longitudinal et où l'ouverture de manchon est formée de façon à ce que les deux bords de côté soient inclinés d'un bas vers un haut.

7. Une protection interlabiale (1) selon n'importe lesquelles des revendications 1 à 6 où la longue zone convexe (3) a une coupe transversale longitudinale triangulaire.

8. Une protection interlabiale (1) selon n'importe lesquelles des revendications 1 à 7, où une zone en coupe transversale longitudinale de la longue zone convexe dans la direction latérale fait au moins 1 cm².

9. Une protection interlabiale (1) telle que revendiquée dans n'importe lesquelles des revendications 1 à 8 où une zone en coupe transversale longitudinale de la longue zone convexe (3) diminue de façon continue à mesure que la zone est prise d'une extrémité à l'autre extrémité suivant la direction longitudinale.

10. Une protection interlabiale (1) selon n'importe lesquelles des revendications 1 à 9 où la protection interlabiale (1) est une protection interlabiale coexistant avec une serviette hygiénique qui est utilisée conjointement avec une serviette hygiénique.

11. Une protection interlabiale (1) selon n'importe lesquelles des revendications 1 à 10 où la protection interlabiale (1) comprend une protection interlabiale pour incontinent destinée à l'incontinence.

12. Une protection interlabiale (1) selon n'importe lesquelles des revendications 1 à 10 où la protection interlabiale comprend une protection interlabiale pour pertes vaginales destinée à absorber des pertes vaginales.
